(19) Europäisches Patentamt / European Patent Office / Office européen des brevets

(11) **EP 2 647 695 A1**

(12) **DEMANDE DE BREVET EUROPEEN**

(43) Date de publication:
**09.10.2013 Bulletin 2013/41**

(51) Int Cl.:
*C12N 1/20* (2006.01)       *C05F 11/08* (2006.01)
*A01N 63/02* (2006.01)       *C05G 3/00* (2006.01)

(21) Numéro de dépôt: **13366002.7**

(22) Date de dépôt: **02.04.2013**

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**
Etats d'extension désignés:
**BA ME**

(30) Priorité: **03.04.2012  FR 1200992**

(71) Demandeur: **Polyor SARL**
**54000 Nancy (FR)**

(72) Inventeur: **Claude, Pierre-Philippe**
**54000 Nancy (FR)**

(54) **Mode de préparation de bouillies inoculantes pulvérisables comprenant des populations bactériennes moyennement halophiles**

(57)     Procédé de préparation de bouillies inoculantes pulvérisables comprenant des populations bactériennes osmotolérantes caractérisé en ce que les bouillies sont réalisées en deux temps ; (i) création dans un premier temps de pré - bouillies plus concentrées en sels fertilisants que les susdites bouillies par un facteur de concentration permettant d'atteindre une milliosmolarité (mOSM) critique responsable de l'induction statique de l'osmotolérance des susdites populations bactériennes a *priori* osmotolérantes, et (ii) abolition dans une deuxième temps et après une certaine période d'induction statique de ce facteur de concentration par dilution des pré - bouillies jusqu'à un volume correspondant au volume préconisé pour de telles bouillies pulvérisables contenant des populations bactériennes osmotolérantes.

$$y = 0,1387x + 0,1909$$
$$R^2 = 0,9963$$

Figure 6

**Description**

**DOMAINE TECHNIQUE DE L'INVENTION**

Microbiologie appliquée à l'agronomie. L'homme de métier, sans nécessairement être microbiologiste, sera attentif à la conservation et la survie d'inocula microbiens destinés à la biofertilisation et/ou au bio-contrôle des cultures viticoles, arboricoles et des grandes cultures. Il est-aussi question de formulation et le conditionnement de préparations bactériennes non sporulantes et/ou particulièrement sujettes à dégradation dans le temps.

**ÉTAT DE LA TECHNIQUE ET PROBLÉMATIQUE**

*Formulation et application d'inocula bactériens par pulvérisation liquide*

Les BFCP (bactéries favorisant la croissance des plantes) colonisent, outre les racines de certaines cultures, les résidus de cultures, les engrais organo- minéraux et/ou les biomasses racinaires résiduelles. Les modes d'action comprennent : (i) la production de sidérophores extracellulaires, (ii) l'antibiose contre des bactéries et des champignons pathogènes, (iii) la production de *phytohormones* (*eg.* auxines), et iv) la solubilisation des phosphates organiques et inorganiques. Or, les BFCP appartiennent à plusieurs genres, y compris *Agrobacterium, Alcaligenes, Arthrobacter, Azotobacter, Bacillus, Cellulomonas, Erwinia, Flavobacterium, Pseudomonas, (Brady) rhizobium, Xanthomonas et Stenotrophomonas.*

L'application de telles BFCP par pulvérisation liquides, au feuillage ou encore au résidus de culture pailleux au sol, est recherchée en agronomie. Elle implique généralement la re-suspension d'un inoculum, solide ou liquide, en milieux aqueux et ainsi la formation d'une "bouillie" directement dans le bac (*in baco*) du pulvérisateur. Lorsque appliquées aux feuillages, de telles préparations auront à affronter des stresses osmotiques du fait de la coprésence de sels fertilisants et/ou de haut taux d'évapotranspiration. En effet, l'évaporation de la solution aqueuse ainsi apportée aux feuillages concentrerant les sels, de tels stresses osmotiques peuvent ainsi détruire ces populations bactériennes, du moins si elles ne sont pas suffisamment osmotolérantes.

De plus, dans la phyllosphère (feuillage), la disponibilité de sucres métabolisables par des bactéries, notamment diazotrophes, est faible, de l'ordre des kg par hectare (Murty *et al.* 1984, Johan *et al.* 2001) . Pour complémenter en substrats glucidiques une activité bactérienne diazotrophe il faudrait apporter plusieurs centaines de kg de sucres pour réduire au plus une vingtaine de kg- N par hectare. Il serait plus opportun d'apporter à un inoculum bactérien foliaire un *agent azoto- nutritionnel* (ANN)- tel qu'un sel d'acide glutamique favorisant l'entrée de l'azote provenant de la dissolution de sels fertilisants dans le cycle de synthèse des protéines (EP10366001.5) . De telles BFCP devront elles aussi affronter des stresses osmotiques bactéricides et/ou bacteriostatiques.

**Osmotolérance induite et halophilie constitutive**

Des osmotolérances à l'égard de ~500 mOSM sont généralement requises pour les BFCP destinées aux applications foliaires (Bonaterrea *et al.* 2005, 2007) ; l'osmolarité de la solution du sol est elle ~50 mOSM (Miller et Wood 1996). La richesse et la diversité bactérienne de la phyllosphère étant limitée (*supra*), la plupart de BFCP candidates viennent donc des sols, et ne seront donc pas *a priori* très halophiles, bien que potentiellement osmotolérantes une fois induites.

Nous devons donc faire ici une distinction entre *halophilie constitutive* et *osmotolérance induite.* Pour faire simple disons que les bactéries halophiles *le sont*, tandis que les bactéries osmotolérantes *le deviennent*. Puisque bon nombre de BFCP ne sont pas foncièrement halophiles (*eg. Pseudomonas, Azotobacter, etc.*), c'est l'induction de leurs osmotolérances qui sera ici la plus utile. En effet, les enzymes de bactéries halophiles non seulement résistent à mais exigent de fortes osmolarités (Sleator et Hill 2000, Goldman *et al.* 1990). Elles abritent de nombreuses charges négatives à leurs surfaces dont la répulsion mutuelle entraîne la dénaturation de l'enzyme à des osmolarités environnement plus habituelles de l'ordre de 500 mM ; l'accumulation cytolytique extraordinaire de cations peut ainsi, en neutralisant ces charges répulsives, préserver le fonctionnement du cytosol. L'halophilie constitutive de certaines BFCP exige donc des concentrations cationiques au delà de celles normalement mesurée in situ dans les sols, voire même la phyllosphère. Mieux faut donc parier sur l'osmotolérance induite de BFCP que moyennement halophiles.

L'osmotolérance bactérienne est connue (Tableau 1). Il existe deux type de valeurs osmolaires ; (i) celles choisies *a priori*, et (ii) celles établies *a posteriori* sur la base d'un indice de survie (*eg.* IC50) ou de croissance (*eg.* MIC ; Purvis *et al.* 2005). Ces deux types de valeurs concordent, une valeur moyenne représentative capable d'induire l'accumulation d'osmolytes compatibles étant de ~500 mOSM. A noter que cette mOSM est 10 à 12 fois plus élevée que celle dans la solution du sol, soit d'environ 50 mOSM (Miller et Wood 1996).. Ces seuils critiques ~500 mOSM ne sont pas nécessairement bactéricides en soi, mais simplement capable d'induire une suraccumulation - par synthèse *de*

*novo* et/ou prélèvement extracellulaire, d'*osmolytes* compatibles. De plus, les délais d'induction de la production d'osmolytes compatibles sont courts, de l'ordre de quelques à plusieurs heures (Tableau 2).

**Tableau 1** : Milli-osmolarité (mOSM ; mmol / L) utilisées par divers expérimentateurs pour induire *activement* et/ou constater l'osmotolérance de diverses bactéries et/ou BFCP.

| milli-osmolarité (mOSM) | soluté | microorganism | commentaires | référence |
|---|---|---|---|---|
| 324 | NaCl | *Synechocystis* | induction expérimental | Hagemann et Erdmann 1994 |
| 648 | NaCl | *Synechocystis* | induction expérimental | Hagemann et Erdmann 1994 |
| 1296 | Sucrose | *Synechocystis* | induction expérimental | Hagemann et Erdmann 1994 |
| 512 | NaCl | *Synechocystis* | induction expérimental | Klähn *et al.* 2010 |
| 450 | NaCl | *Synechocystis* | induction expérimental | Ferjanni *et al.*2003 |
| 385 | NaCl | *Stenotrophomonas rhizophilia* | induction expérimental | Hagemann *et al.* 2008 |
| 512 | NaCl | *Stenotrophomonas rhizophilia* | induction expérimental | Wolf *et al.* 2002 |
| 500 | NaCl | *Azotobacter* | induction expérimental | Madkour *et al.* 1990 |
| 500 | NaCl | *Azospirillum* | induction expérimental | Madkour *et al* 1990 |
| 128 | NaCl | *Pseudomonas syringae* | induction expérimental | Kurz *et al.* 2010 |
| 256 | NaCl | *Pseudomonas syringae* | induction expérimental | Kurz *et al.* 2010 |
| 385 | NaCl | *Pseudomonas syringae* | induction expérimental | Kurz *et al.* 2010 |
| 500 | NaCl | *Klebsiella* | induction expérimental | Madkour *et al.* 1990 |
| 500 | NaCl | *Erwina chrysanthemi* | induction expérimental | Gouesbet *et al.* 1996 |
| 500 | NaCl | *Erwina chrysanthemi* | induction expérimental | Goude *et al.* 2004 |
| *205* | KCl | *E. coli* | constaté selon IC50 | Purvis *et al.* 2005 |
| *291* | KH2PO4 | *E. coli* | constaté selon IC50 | Purvis *et al.* 2005 |
| *182* | NaCl | *E. coli* | constaté selon IC50 | Purvis *et al.* 2005 |
| *325* | Arabinose | *E. coli* | constaté selon IC50 | Purvis *et al.* 2005 |
| *314* | Glucose | *E. coli* | constaté selon IC50 | Purvis *et al.* 2005 |
| *324* | Mannose | *E. coli* | constaté selon IC50 | Purvis *et al.* 2005 |
| *141* | Xylose | *E. coli* | constaté selon IC50 | Purvis *et al.* 2005 |
| 469 | KCl | *E. coli* | constaté selon MIC | Purvis *et al.* 2005 |
| 496 | KH2PO4 | *E. coli* | constaté selon MIC | Purvis *et al.* 2005 |
| 463 | NaCl | *E. coli* | constaté selon MIC | Purvis *et al.* 2005 |
| 386 | Arabinose | *E. coli* | constaté selon MIC | Purvis *et al.* 2005 |
| 488 | Glucose | *E. coli* | constaté selon MIC | Purvis *et al.* 2005 |
| 469 | Mannose | *E. coli* | constaté selon MIC | Purvis *et al.* 2005 |
| 164 | Xylose | *E. coli* | constaté selon MIC | Purvis et *al.* 2005 |

(suite)

| milli-osmolarité (mOSM) | soluté | microorganism | commentaires | référence |
|---|---|---|---|---|
| **430** | **na** | **na** | **MOYENNE** | **na** |
| ~ 50 | sels divers | microflore du sol | condition *in pedo* | Miller et Wood 1996 |

**TABLEAU 2** : Temps (délais) d'induction de la production d'*osmolytes* compatibles chez diverses bactéries

| temps d'incubation | délais d'induction | milli-osmolarité (mOSM) | soluté | osmolyte prédominant | microorganism | référence |
|---|---|---|---|---|---|---|
| 120 minutes | 4 à 5 minutes | 324 | NaCl | glucosyl-glycérol (GG) | *Synechocystes* | Hagemann et Erdmann 1994 |
| 120 minutes | 4 à 5 minutes | 648 | NaCl | glucosyl-glycérol (GG) | *Synechocystes* | Hagemann et Erdmann 1994 |
| 120 minutes | 4 à 5 minutes | 1296 | Sucrose | glucosyl-glycérol (GG) | *Synechocystes* | Hagemann et Erdmann 1994 |
| 12 heures | 1 heure | 512 | NaCl | glucosyl-glycérol (GG) | *Synechocystes* | Klähn *et al.* 2010 |
| 72 heures | 10 heures | 450 | NaCl | glucosyl-glycérol (GG) | *Synechocystes* | Ferjanni *et al.* 2003 |
| 32 heures | 4 heures | 385 | NaCl | glucosyl-glycérol (GG) | *Stenotrophomonas rhizophilia* | Hagemann *et al.* 2008 |
| selon API ? | selon API ? | 512 | NaCl | glucosyl-glycérol (GG) | *Stenotrophomonas rhizophilia* | Wolf *et al.* 2002 |
| 18 heures | (18 heures ?) | 500 | NaCl | tréhalose (TRE) | *Azotobacter* | Madkour *et al.* 1990 |
| 18 heures | (18 heures ?) | 500 | NaCl | proline (PRO), glutamate (GLU) | *Klebsiella* | Madkour *et al.* 1990 |
| 30 heures | (30 heures ?) | 500 | NaCl | proline (PRO), glutamate (GLU) | *Azospirillum* | Madkour et al. 1990 |
| 72 heures | (72 heures ?) | 128 | NaCl | NAGGN | *Pseudomonas syringae* | Kurz *et al.* 2010 |
| 72 heures | (72 heures ?) | 256 | NaCl | NAGGN | *Pseudomonas syringae* | Kurz *et al.* 2010 |
| 72 heures | (72 heures ?) | 385 | NaCl | NAGGN | *Pseudomonas syringae* | Kurz *et al.* 2010 |
| 15 heures | 5 heures | 500 | NaCl | divers (PRO, bétaine, ectoine, etc.) | *Erwina chrysanthemi* | Gouesbet *et al.* 1996 |
| 28 heures | 1 à 25 heures | 500 | NaCl | glucosyl-glycérate (GGate) | *Erwina chrysanthemi* | Goude *et al.* 2004 |

(suite)

| temps d'incubation | délais d'induction | milli-osmolarité (mOSM) | soluté | osmolyte prédominant | microorganism | référence |
|---|---|---|---|---|---|---|
| 30 heures | 5-10 heures | 205 | KCl | tréhalose (TRE) | *E. coli* | Purvis *et al.* 2005 |
| 30 heures | 5-10 heures | 291 | KH2PO4 | tréhalose (TRE) | *E. coli* | Purvis *et al.* 2005 |
| 30 heures | 5-10 heures | 182 | NaCl | tréhalose (TRE) | *E. coli* | Purvis *et al.* 2005 |
| 30 heures | 5-10 heures | 325 | Arabinose | tréhalose (TRE) | *E. coli* | Purvis *et al.* 2005 |
| 30 heures | 5-10 heures | 314 | Glucose | tréhalose (TRE) | *E. coli* | Purvis *et al.* 2005 |
| 30 heures | 5-10 heures | 324 | Mannose | tréhalose (TRE) | *E. coli* | Purvis *et al.* 2005 |
| 30 heures | 5-10 heures | 141 | Xylose | tréhalose (TRE) | *E. coli* | Purvis *et al.* 2005 |
| 30 heures | 5-10 heures | 469 | KCl | tréhalose (TRE) | *E. coli* | Purvis *et al.* 2005 |
| 30 heures | 5-10 heures | 496 | KH2PO4 | tréhalose (TRE) | *E. coli* | Purvis *et al.* 2005 |
| 30 heures | 5-10 heures | 463 | NaCl | tréhalose (TRE) | *E. coli* | Purvis *et al.* 2005 |
| 30 heures | 5-10 heures | 386 | Arabinose | tréhalose (TRE) | *E. coli* | Purvis *et al.* 2005 |
| 30 heures | 5-10 heures | 488 | Glucose | tréhalose (TRE) | *E. coli* | Purvis *et al.* 2005 |
| 30 heures | 5-10 heures | 469 | Mannose | tréhalose (TRE) | *E. coli* | Purvis *et al.* 2005 |
| 30 heures | 5-10 heures | 164 | Xylose | tréhalose (TRE) | *E. coli* | Purvis *et al.* 2005 |

Or, ces accumulations d'osmolytes compatibles s'accomplissent toujours en pleine croissance des populations bactériennes, voire le plus souvent en fin de phase de croissance exponentielle ; on parle ici d'*induction dynamique* de l'osmotolérance. Il ne s'agit donc pas d'acclimatation, mais bien de conditionnement métabolique. L'osmotolérance induite demeure donc aux yeux de l'homme du métier un phénomène essentiellement lié à la croissance en milieux de culture.

Pourtant, l'*induction statique* de l'osmotolérance existe et peut contribuer à rendre plus résistantes les biomasses bactériennes pulvérisables. En effet, les travaux de Cayley *et al.* (1991, 2000) indiquent qu'un phénomène d'*encombrement moléculaire* (*molecular crowding* ; Ellis 2001, Spitzer 2011) dans les cytosol de bactéries osmo- stressées résulte d'un volume cellulaire (Vc uL / mg de protéine) plus petit ; les interactions protéines: ADN sont donc favorisées et contrefont d'éventuels effets néfastes liés à une surconcentration cationique. Cela dit, l'encombrement moléculaire à hautes pressions osmotiques environnantes n'est pas explicitement valorisé en agronomie, mais ne sert qu'à expliquer le métabolisme soutenue de certaines bactéries malgré de telles pressions osmotiques environnantes (Ellis 2001, Zimmermann et Trach 1991, Spitzer 2011) .

## DIVULGATION DE L'INVENTION

*Problème technique aperçu*

La phyllosphère - du fait d'une évapotranspiration importante favorisant l'accumulation de sels résiduels, est un environnement plutôt *osmogène* ; de tels stress osmotiques sont généralement suffisants pour nuire au ou arrêter le fonctionnement desdites BFCP, y comprises celles provenant des sols. Ces stresses osmotiques sont d'autant plus nuisibles si un sel fertilisant est apporté conjointement au feuillage.

Or, en réponse à ces stresses osmotiques il eut été possible en principe de simplement apporter aux feuillages des bactéries expressément et foncièrement *osmotolérantes*, voire halophiles. Or, des bactéries halophiles, en raison de leurs adaptation à des environnement très osmogènes, nécessitent des potentiels hydriques et des pression osmotique environnant peu compatibles avec le fonctionnement habituel de BFCP, voire des phyllosphères qu'elles doivent habiter. Les populations bactériennes osmotolérantes, et donc ici que moyennement halophiles, doivent elles être induites à l'être lors de leurs croissance. De plus, cette osmotolérance une fois induite n'est pas nécessairement constitutive, et un certain délai une fois le stress osmotique *in folio* (i.e. sur/ dans le feuillage) perçue par les bactéries. Or entre temps, ledit stress osmotique, particulièrement important lors de l'évaporation de la solution aqueuse peut lui s'avérer franchement bactéricide. Il faut donc recourir à une induction post- croissance afin de maximiser, ou même assurer *in folio* la survie desdites BFCP en présences de sels fertilisants NP.

Cela dit, les BFCP ainsi les plus osmotolérantes ont aussi des volumes cellulaires (Vc ; uL/mg de protéine bactérienne) réduits en raison d'un certain encombrement moléculaire. Or, ces bactéries, en raison de ces Vc *comprimés* sont ainsi d'autant plus sujettes à des stresses *hypo - osmotiques,* notamment si elles doivent être immergées *in baco* dans des bouillies pulvérisables d'osmolarités quelconques.

L'osmotolérance acquise par induction dynamique via l'imposition d'une osmolarité quelconque lors de la croissance des bactéries (*osmolarité de croissance* ; OSMc) devrait dicter l'*osmolarité de plasmolyse* (OSMp) en dessous de laquelle lesdites bactéries *exploseront* en raison d'une trop forte pression intracellulaire ($\pi_i$). C'est cet ajustement OSMc ≈ OSMp qui n'est pas à ce jour explicitement pris en compte par l'homme du métier car il implique le plus souvent la formation d'une *pré - bouillie, in baco,* plus concentrée *a priori* perçue comme doublement préjudiciable à la survie desdites bactéries.

*Solution technique proposée*

La solution technique consiste à ajuster, précisément, l'osmolarité de la bouillie, *in baco,* en fonction de l'osmolarité de croissance (OSMc) à la quelle les bactéries ont été produites. Cela implique la formation, transitoire sur une période, par exemple, de 8 à 12 heures, d'une pré - bouillie le plus souvent plus concentrée que la bouillie pulvérisable ; il fallut donc prévoir et jauger la dilution de cette pré - bouillie "sur - concentrée", en sorte, afin de respecter une OSMp calculée qu'il faut donc explicitement respecter. La solution technique proposée consiste donc à placer des BFCP, avantageusement chitinolytiques et/ou fongistatiques, au sein de pré - bouillies dont l'osmolarité préétablie aux environs d'un certain seuil critique, ici et à titre d'exemple le plus avantageux ~500 mOSM, induira une osmotolérance acquise lors de la croissance de ces populations bactériennes.

Cette solution technique implique la mise en contacte directe au sein de ladite pré - bouillie desdites BFCP et de sels fertilisants, mise en contacte sels/bactéries pourtant contraire aux habitudes de l'homme de métiers, d'autant plus que ces pré - bouilles sont expressément sur - concentrées, ou plus exactement sous - diluées (*infra*). Le coeur de l'invention est donc une certaine « pré - bouillie » *in baco* concentrant transitoirement les sels fertilisants normalement présents dans la bouillie pulvérisable finale par un certain facteur de concentration selon le poids moléculaire des principaux constituants, avantageusement des sels azotée et/ou agents azoto-nutritionnels, la concentration de ces constituants, la dose-hectare, ainsi que le volume pulvérisable finalement préconisé. Or, ce volume final de la bouille sera lui aussi précisé en fonction le l'osmolarité de croissance (OSMc) desdites populations bactériennes.

Concrètement, il s'agit d'un procédé de préparation de bouillies inoculantes pulvérisables comprenant des populations bactériennes osmotolérantes caractérisé en ce que les bouillies sont réalisées en deux temps ; (i) création dans un premier temps de pré- bouillies plus concentrées en sels fertilisants que les susdites bouillies par un facteur de concentration permettant d'atteindre une milliosmolarité (mOSM) critique responsable de l'induction statique de l'osmotolérance des susdites populations bactériennes *a priori* osmotolérantes, et (ii) abolition dans une deuxième temps et après une certaine période d'induction statique de ce facteur de concentration par dilution des pré- bouillies jusqu'à un volume correspondant au volume préconisé pour de telles bouillies pulvérisables contenant des populations bactériennes osmotolérantes. Les populations bactériennes sont constituées d'espèces bactériennes Gram négatives (Gram[-]) et/ou Gram positives (Gram[+]) .

La période d'*induction statique* est comprise entre 6 et 24 heures, plus particulièrement entre 8 et 18 heures, et plus usuellement d'environs 12 heures.

La milliosmolarité (mOSM) critique de la pré - bouillie concentrée est comprise entre 330 et 1 250, et avantageusement aux alentours de 500. À titre illustratif, les volumes (L) de telles pré - bouillies permettant d'assurer mOSM d'approximativement 500 mM, facteur d'induction statique de l'osmotolérance des bactéries osmotolérantes concernées, sont présentés au Tableau 4.

La dilution desdites pré - bouillies est effectuée par l'apport d'un volume aqueux fonction de la dose hectare (L/ha) du produit, du poids moléculaire des sels fertilisants le constituant (g/mole), et leurs concentrations dans le produit (g-sel/L) respectant une mOSM minimale de la bouillie fonction de l'osmolarité de croissance (OSMc) appliquée lors production des populations bactériennes en milieux liquides et/ou solide. La mOSM minimale fonction de l'osmolarité de croissance (OSMc) appliquée lors production des populations bactériennes en milieux liquides et/ou solide est elle fonction de l'osmolarité de plasmolyse (OSMp) de ces populations bactériennes. Enfin, l'osmolarité de plasmolyse (OSMp) de ces populations bactériennes est elle donnée par la le paramètre $\alpha_2$ (mOSM) de la relation décrivant le volume cellulaire, $V_c$ (uL/mg de protéine bactérienne), desdites bactéries en fonction d'OSMPp, à savoir **[$V_c = \alpha_1 / (OSMp + \alpha_2) + V_{c,min}$,]** où $V_{c,min}$ et le volume cellulaire minimal atteint en fonction d'OSMp et $\alpha_1$ est un autre paramètre empirique (uL/mg de protéine bactérienne).

Les populations bactériennes *a priori* osmotolérantes sont produites par fermentation liquide ou solide de milli-osmolarités de croissance (OSMc) comprises entre 50 et 1 000, plus particulièrement entre 200 et 800, et avantageusement entre 300 et 600. Le facteur de concentration à abolir est lui compris entre 3,8 et 113 et cela selon la concentration (g-sel/L) des sels fertilisant, le poids moléculaire (PM) de ceux-ci et la quantité de produit préconisé par hectare (i.e. dose-hectare en g, Kg ou L par hectare), voire - le cas échéant, l'osmolarité de la fraction soluble de la formulation solide et/ou liquide, de ladite préparation bactérienne. La milli-osmolarité (mOSM) de la bouillie une fois ledit facteur de concentration aboli par dilution aqueuse ne doit pas elle être inférieure à des valeurs comprises entre 175 et 360, plus avantageusement entre 190 et 330, notamment dans le cas de bactéries produites en présence d'OSMc (omolarités de croissance) les plus élevées, soit ici de l'ordre de 1,00.

Les populations bactériennes sont avantageusement chitinolytiques, et/ou proviennent de familles taxonomiques choisies parmi un groupe comprenant les *Xanthomonadaceae* et les *Pseudomonadaceae*, voire du genre *Stenotrophomonas.* Ces populations bactériennes *a priori* osmotolérantes produisent comme osmolyte compatible du glucosylglycérol (GG) et/ou du glucosyl-glycerate (GGate). A titre d'exemple, *Stenotrophomonas maltophilia* (Crossman *et al.* 2008), précédemment Pseudomomonas *maltophilia* et/ou *Xanthomonas maltophilia* est ubiquitaire dans les sols, et taxonomiquement proche des *Azotobacteracea.* De nombreuses espèces ont été répertoriées ; *S. terrea, S. humi, S. rhizophilia, S. dokdonesis, etc.* (Hagemann *et al.* 2008, Wolf *et al.* 2002, Heylen *et al.* 2007, Yoon *et al.* 2008). Parce que certaines espèces de sont chitinolytiques (Metcalfe *et al.* 2002a, 2002b ; Kobayashi *et al.* 2002), *Stenotrophomonas* est parfois préconisée pour le biocontrôle de maladies fongiques foliaires *Poaceae* causées par *Rhizoctonia solani* (Giesler et Yuen 1998), ou encore *Magnaprothe poae* (Kobayashi *et al.* 1995). Or, l'efficacité de telles applications n'est pas très bonne du fait d'osmotolérances insuffisantes. Pourtant, certaines *Stenotrophomonas* peuvent être *induites* à produire un *osmolyte compatible ;* le glycosyl glycérol (Marin *et al.* 2002) voire d'autres osmolytes.

*Principaux avantages, et activité inventive*

L'invention permet de mieux réconcilier l'application foliaire de sels fertilisants et BFCP, et donc du coup fertilisation foliaire et régie phytosanitaire des cultures, et cela sans surcharge de travail pour l'utilisateur puisque les deux opérations peuvent être combinées en une seule.

Cette (sur)concentration de sels combinée à des préparations bactériennes est toute sauf évidente pour l'homme du métier. La hauteur inventive de cette solution technique est donc appréciable dans la préconisation d'une « pré - bouillie » *in baco*, sur-concentrée en sorte par rapport à la bouillie pulvérisable *in fine.* Pour le microbiologiste, mais non pour l'agronome et/ou l'agriculteur, cette sur-concentration peut effectivement induire une certaine osmotolérance des BFCP ; une fois cette induction terminée le facteur de concentration est aboli lors du rétablissement du volume préconisé pour la bouillie pulvérisable. L'homme du métier ne peut pas cependant concevoir qu'une telle exposition à un quelconque stress en soi bien supérieur à l'osmolarité du sol - soit généralement de l'ordre de 50 mOSM (Miller et Wood 1996), puisse être recherchée et/ou comporter en soi un avantage technique en agronomie. Il cherchera plutôt à préserver au maximum la survie de ces microorganismes jusqu'à leurs application au champ ; cette préservation doit donc à ses yeux exclure au tant que possible un tel stress osmotique.

## BREVE DESCRIPTION DES DESSINS ET FIGURES

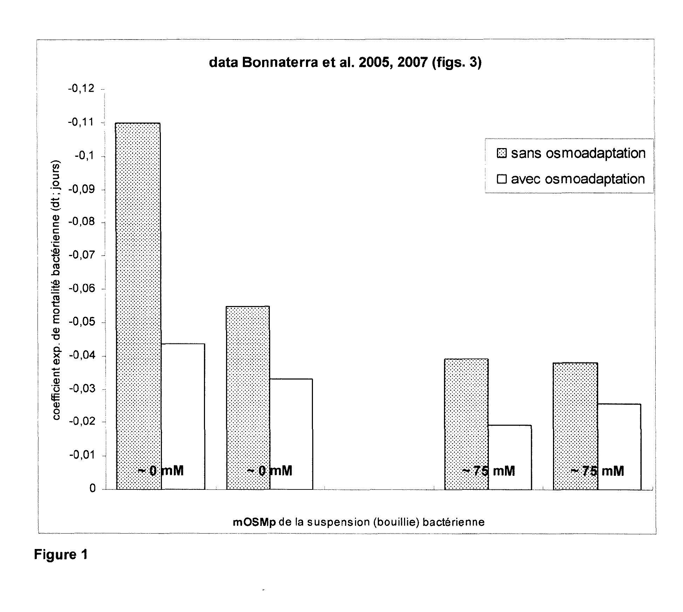

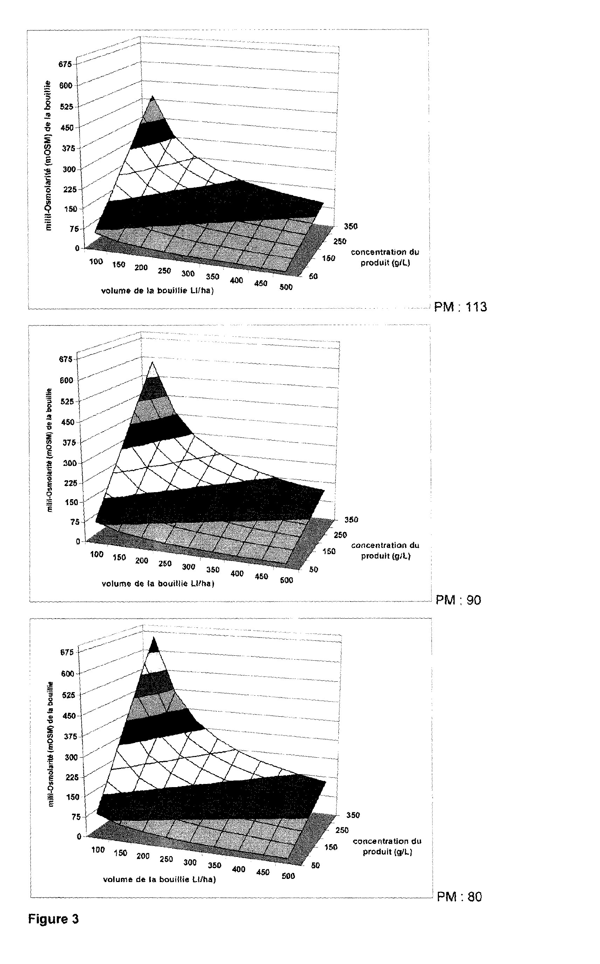

**Figure 1** : Les coefficients exponentiels de mortalités en fonction de la durée de conditions propices au dessèchement de bactéries Gram⁻ obtenues de cultures avec et sans osmo- adaptation en présence de 0, 5 ou 0, 7 M-NaCl (données de Bonaterra *et al.* 2005 et 2007, figures 3) . Quatre expérimentations sont décrites ; les deux premières comprennent des bactéries osmo- adaptées à 0, 5 M re- suspendues dans le l'eau distillée (~ 0 mM) ; le deux dernières comprennent des bactéries osmo- adaptées à 0, 7 M re- suspendues dans une solution dites de Ringer diluée (~ 75 mM) . A noter donc un diminution du taux de mortalité avec osmo-adaptation, mais aussi l'effet bénéfique sur la survie desdites bactéries d'une certaine osmolarité (~ 75 mM) de la suspension (bouillie) .

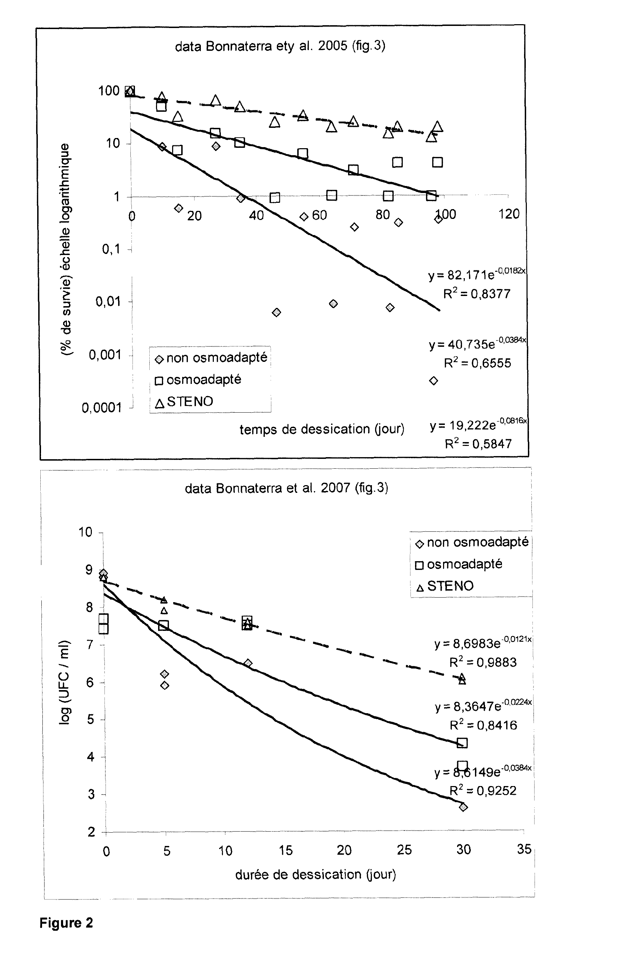

**Figure 2** : Résumé graphique des données de Bonaterra *et al.* 2005 et 2007 ayant servi à établir les coefficients exponentiels à la Figure 1 ; les deux expérimentations comprenant des bactéries osmo- adaptées à 0, 5 M- NaCl sont regroupées (figure du haut ; data Bonaterra 2005), ainsi que les celles comprenant des bactéries osmo- adaptées à 0, 7 M- NaCl (figure du bas ; data Bonaterra 2007) . Il s'agit donc de courbe de survie (%) en fonction du temps de dessèchement (*desiccation*) de bactéries osmo- adaptées ou non. A titre illustratif j'ai placé une courbe (en pointillé) escomptant la survie de telles bactéries dont l'osmolarité de la suspension (bouillie) aurait été ajusté en fonction de leurs l'osmolarités de croissance (OSMc) au sens de la présente invention.

**Figure 3** : Milli- osrmolarité (mOSM) des bouillies (*eg.* 15 L- produit / ha) selon le poids moléculaire (PM) des sels constituants, le volume préconisé de la bouillie, ainsi que la concentration (g/L) du produit. A noter que bon nombre de ces mOSM sont inférieurs à 500 mOSM ; le bouillies concernées devront- au sens de la présente invention, être concentrées par un facteur. C'est ce facteur de concentration qui devra être aboli au moment de l'utilisation de la bouillie.

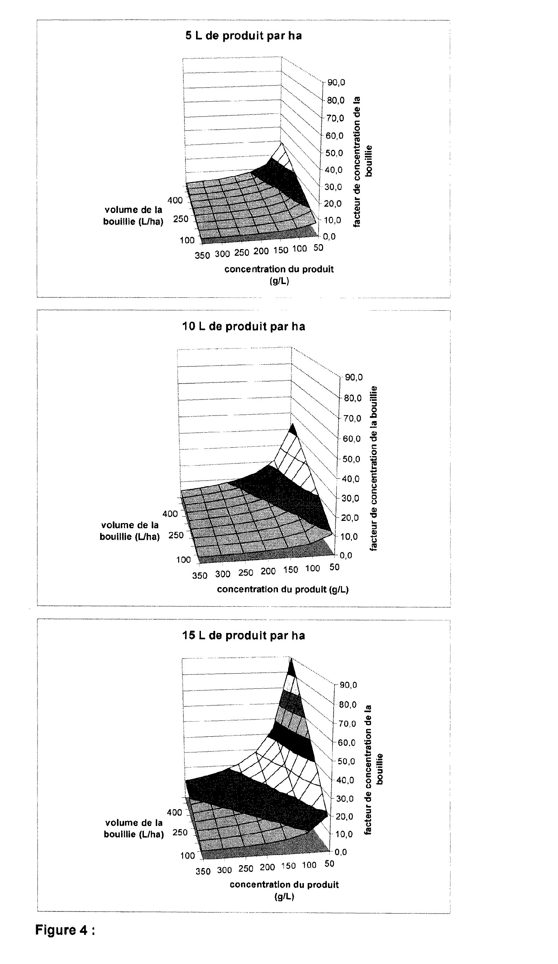

**Figure 4** : Facteur de concentration appliqué à la pré- bouillie et à abolir au moment de l'utilisation de la bouillie ; c'est ce facteur de concentration qui permet d'assurer une mOSM de 500 propice à l'induction statique de l'osmotolérance des souches bactériennes (*eg.* PM = 90 (urée formaldéhyde ; Azo- Speed™, AzoFol SR™) .

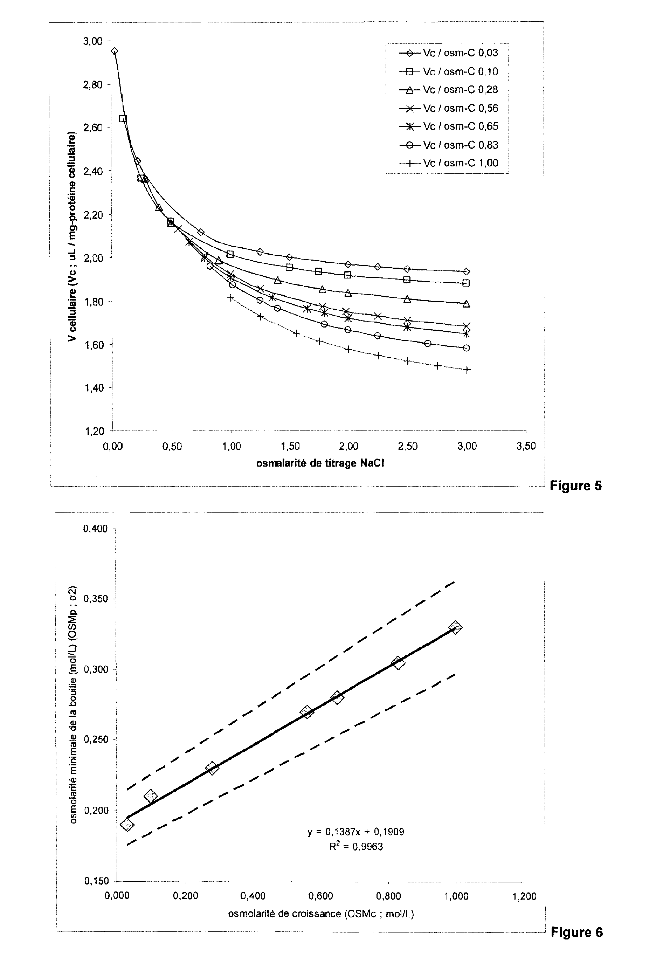

**Figure 5** : Modélisation du volume cellulaire des bactéries selon les osmolarités de plasmolyses (OSMp) . Ces osmolarités de plasmolyses sont établies par *titrage osmotique* selon Cayley *et al.* (1991, 2000) . A noter que cette décroissance de Vc est de plus en plus abrupte pour les osmolarités de croissance (OSMc) plus importantes, dénotant ainsi une certaine osmoadaptation associée de facto à un encombrement moléculaire grandissant ; cette encombrement moléculaire permets à la cellule de maintenir son activité métabolique- ou du moins adénique, malgré de forte concentration cytoplasmique de cations (voir texte) .

**Figure 6** : Osmolarités minima de la bouillie telles qu'inférée de la variable paramétrique $\alpha 2$ pour le calcul de l'osmolarité de plasmolyses (OSMp) selon l'osmolarité de croissance (OSMc) de bactéries osmotolérantes Gram[-] ; Tableau 6. A mesure que progresse cette OSMc effective lors de la production des biomasses bactériennes de l'inoculum, l'osmolarité minimale de la bouillie doit être égale à OSMp. Les osmolarités minima de la bouillie de bactéries osmotolérantes Gram[+], en principe plus halophiles, sont elles décrites par une fonction dont la courbe (pointillée) est légèrement déplacée vers le haut (+10%) . Idem pour ce qui concernerait des bactéries osmotolérantes moins halophiles, les osmolarités minima de la bouillie serait elles décrites par une courbe (pointillée) déplacée vers le bas (- 10%) .

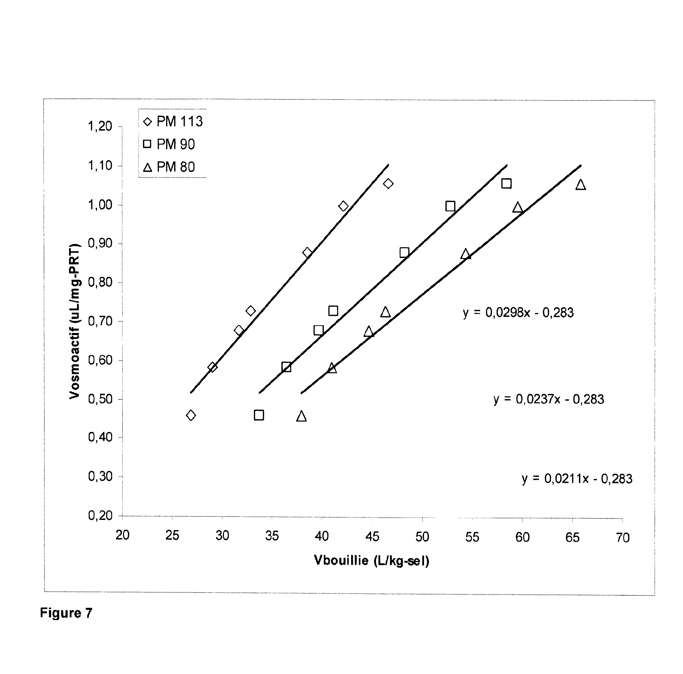

**Figure 7** : Représentation graphique de la relation entre les volumes des bouillies ($V_{bouillie}$ ; L/kg- sel) pulvérisables contenant des sels fertilisants et dont l'osmolarité est adaptée à celles des cytosols bactériens quelles contiennent, et les volumes osmoactifs ($V_{osmoactif}$ ; uL/mg- protéine microbienne) desdits cytosols microbiens, et plus particulièrement bactériens. Trois courbes sont proposées pour trois poids moléculaires (PM ; g/mol) desdits sels. A noter que ces relations sont foncièrement linéaires et indépendantes de la concentration en sel (g- sel/L) et de la dose- hectare de tels produits solubles (L de concentré / ha) . A noter aussi que le caractère osmogène des sels est donc de ~33 à ~50 fois plus important que celui des soluté des cytosols microbiens rapporté ici sur la base de leurs teneurs en protéines bactériennes.

## MODE DE REALISATION PREFERE DE L'INVENTION

Les bactéries sont avantageusement destinées aux feuillages de cultures viticoles, arboricoles, céréalières et/ou herbacées. Elles sont mélangées en milieux aqueux à des sels fertilisants osmogènes, avantageusement au sein d'un bac d'un pulvérisateur. La milli-osmolarité (mOSM) de la pré-bouillie concentrée ainsi obtenue est avantageusement ~500 mOSM. Le stress osmotique ainsi provoqué permettra aux bactéries, avantageusement des BFCP, d'activer leurs osmotolérance préalablement acquises lors de leurs en présence d'osmolarité de croissance (OSMc) particulières. Ces bactéries peuvent ainsi être mises en contacte avec des feuillages, mais aussi avec des racines, des semences, voire des résidus de culture au sol.

Au Tableau 3, les concentrations en g par litre des bouillies pulvérisables selon la teneur en sel et la dose-hectare de différents produits fertilisant pour application foliaire (*eg.* Azo-Speed™, AzoFol-SR™, Fertigofol 313™; Agronutrition SAS, 31390, Carbonne France), ainsi que les différents volumes de la bouillie pulvérisable tels que préconisés par le fabricant. En fonction des poids moléculaire (PM ; g/mol) des principaux constituants de tels produits, nous pouvons calculer la millio-smolarité (mOSM) de ces bouillies (Figure 3), ainsi que le volume d'une éventuelle pré - bouillie elle nécessairement ~500 mOSM tel que préconisé ici (Tableau 4).

A noter qu'il s'agit ici de d'osmolarité théorique et non de l'*osmolaité* mesurée ; cette dernière étant généralement inférieure du fait de l'interaction intermoléculaire au sein de tels mélanges. A noter aussi que pour une concentration donnée, les valeurs mOSM vont décroître en fonction de la diminution du PM en raison de la plus grande activité osmotique des plus petites molécules.

Du coup, on peut aussi calculer le *facteur de concentration* à appliquer à cette bouillie pulvérisable pour obtenir la pré-bouillie plus osmogène à hauteur d'environ 500 mOSM (Figure 4). Concrètement, il s'agit simplement de placer dans le bac du pulvérisateur les volumes d'eau préconisés au Tableau 4. Ces volumes sont fonction de la teneur en g-sel par L du produit, la dose-hectare de ce produit par hectare et le poids moléculaire de l'ensemble des constituants osmogène dudit produit, y compris le cas échéant d'éventuel co-formulants (*infra*). En complétant, avantageusement le lendemain matin, le volume de la bouillie pulvérisable - soit généralement de l'ordre de 200 à 500 L par hectare, le facteur de concentration sera aboli, et la pulvérisation pourra aller de l'avant.

**Tableau 3 :** Concentration (g / L) de bouillie pulvérisable au champ selon le nombre de L-bouillie / ha préconisés, la concentration du produit en g-sel / L ainsi que le volume (L) de la bouillie par hectare. Selon le poids moléculaire (PM ; g/mole) des principaux constituant du produit, la mili - osmolarité (mOSM) de la bouillie sera plus ou moins importante (Voir à la Figure 1 pour une illustration de ce fait pour le cas de 15 L-produit / ha, le même schéma étant applicable aux autres cas avec plus ou moins de L-produit par hectare).

| L-produit/ha = 5 | L-bouillie/ha | | | | | | | | |
|---|---|---|---|---|---|---|---|---|---|
| g-sel/L | 100 | 150 | 200 | 250 | 300 | 350 | 400 | 450 | 500 |
| 50 | 2,50 | 1,67 | 1,25 | 1,00 | 0,83 | 0,71 | 0,63 | 0,56 | 0,50 |
| 100 | 5,00 | 3,33 | 2,50 | 2,00 | 1,67 | 1,43 | 1,25 | 1,11 | 1,00 |
| 150 | 7,50 | 5,00 | 3,75 | 3,00 | 2,50 | 2,14 | 1,88 | 1,67 | 1,50 |
| 200 | 10,00 | 6,67 | 5,00 | 4,00 | 3,33 | 2,86 | 2,50 | 2,22 | 2,00 |
| 250 | 12,50 | 8,33 | 6,25 | 5,00 | 4,17 | 3,57 | 3,13 | 2,78 | 2,50 |
| 300 | 15,00 | 10,00 | 7,50 | 6,00 | 5,00 | 4,29 | 3,75 | 3,33 | 3,00 |
| 350 | 17,50 | 11,67 | 8,75 | 7,00 | 5,83 | 5,00 | 4,38 | 3,89 | 3,50 |

| L-produit/ha = 10 | L-bouillie/ha | | | | | | | | |
|---|---|---|---|---|---|---|---|---|---|
| g-sel/L | 100 | 150 | 200 | 250 | 300 | 350 | 400 | 450 | 500 |
| 50 | 5,00 | 3,33 | 2,50 | 2,00 | 1,67 | 1,43 | 1,25 | 1,11 | 1,00 |
| 100 | 10,00 | 6,67 | 5,00 | 4,00 | 3,33 | 2,86 | 2,50 | 2,22 | 2,00 |
| 150 | 15,00 | 10,00 | 7,50 | 6,00 | 5,00 | 4,29 | 3,75 | 3,33 | 3,00 |
| 200 | 20,00 | 13,33 | 10,00 | 8,00 | 6,67 | 5,71 | 5,00 | 4,44 | 4,00 |
| 250 | 25,00 | 16,67 | 12,50 | 10,00 | 8,33 | 7,14 | 6,25 | 5,56 | 5,00 |
| 300 | 30,00 | 20,00 | 15,00 | 12,00 | 10,00 | 8,57 | 7,50 | 6,67 | 6,00 |
| 350 | 35,00 | 23,33 | 17,50 | 14,00 | 11,67 | 10,00 | 8,75 | 7,78 | 7,00 |

| L-produit/ha = 15 | L-bouillie/ha | | | | | | | | |
|---|---|---|---|---|---|---|---|---|---|
| g-sel/L | 100 | 150 | 200 | 250 | 300 | 350 | 400 | 450 | 500 |
| 50 | 7,50 | 5,00 | 3,75 | 3,00 | 2,50 | 2,14 | 1,88 | 1,67 | 1,50 |
| 100 | 15,00 | 10,00 | 7,50 | 6,00 | 5,00 | 4,29 | 3,75 | 3,33 | 3,00 |

(suite)

| L-produit/ha = 15 | L-bouillie/ha | | | | | | | | |
|---|---|---|---|---|---|---|---|---|---|
| g-sel/L | 100 | 150 | 200 | 250 | 300 | 350 | 400 | 450 | 500 |
| 150 | 22,50 | 15,00 | 11,25 | 9,00 | 7,50 | 6,43 | 5,63 | 5,00 | 4,50 |
| 200 | 30,00 | 20,00 | 15,00 | 12,00 | 10,00 | 8,57 | 7,50 | 6,67 | 6,00 |
| 250 | 37,50 | 25,00 | 18,75 | 15,00 | 12,50 | 10,71 | 9,38 | 8,33 | 7,50 |
| 300 | 45,00 | 30,00 | 22,50 | 18,00 | 15,00 | 12,86 | 11,25 | 10,00 | 9,00 |
| 350 | 52,50 | 35,00 | 26,25 | 21,00 | 17,50 | 15,00 | 13,13 | 11,67 | 10,50 |

Cette abolition du facteur de concentration le lendemain (12 heures) n'est pas arbitraire ; ce délais s'appui sur une revue de la littérature (Tableau 2). Les volumes au Tableau 4 sont à réaliser *in baco* environs 12 heures - la veille, avant la pulvérisation de la bouillie au champ.

Par rapport à la bouillie pulvérisable, cette pré - bouillie est donc plus concentrée par un certain facteur de concentration. Les volumes de la bouillie, par opposition à ceux de la pré - bouillie au Tableau 4, à ne pas dépasser au moment de l'abolition dudit facteur de concentration peu avant sa pulvérisation liquide sont rapportés aux Tableaux 5-a, 5b et 5c selon les différents poids moléculaire des produits à base de sels fertilisants choisis ici à titre d'exemple. Il s'agit ainsi d'éviter des conditions hypo - osmotiques aptes à faire exploser les bactéries expressément induites à être osmotolérantes en présence d'OSMc relativement élevées.

Tableau 4 : Volume (L) de la pré-bouillie permettant d'assurer une milli-osmolarité (mOSM) d'approximativement 500 mMole / L (500 mOSM) facteur d'induction statique de l'osmotolérance des bactéries osmotolérantes concernées.

| L de produit / hectare = 5 | Poids moléculaire (PM ; g/mole) des principaux sels | | |
|---|---|---|---|
| g-sel / L de produit | 113 | 90 | 80 |
| 50 | 4,4 | 5,6 | 6,3 |
| 100 | 8,8 | 11,1 | 12,5 |
| 150 | 13,3 | 16,7 | 18,8 |
| 200 | 17,7 | 22,2 | 25,0 |
| 250 | 22,1 | 27,8 | 31,3 |
| 300 | 26,5 | 33,3 | 37,5 |
| 350 | 31,0 | 38,9 | 43,8 |

| L de produit / hectare = 10 | Poids moléculaire (PM ; g/mole) des principaux sels | | |
|---|---|---|---|
| g-sel / L de produit | 113 | 90 | 80 |
| 50 | 8,8 | 11,1 | 12,5 |
| 100 | 17,7 | 22,2 | 25,0 |
| 150 | 26,5 | 33,3 | 37,5 |
| 200 | 35,4 | 44,4 | 50,0 |
| 250 | 44,2 | 55,6 | 62,5 |
| 300 | 53,1 | 66,7 | 75,0 |
| 350 | 61,9 | 77,8 | 87,5 |

(suite)

| L de produit / hectare = 15 | Poids moléculaire (PM ; g/mole) des principaux sels | | |
|---|---|---|---|
| g-sel / L de produit | 113 | 90 | 80 |
| 50 | 13,3 | 16,7 | 18,8 |
| 100 | 26,5 | 33,3 | 37,5 |
| 150 | 39,8 | 50,0 | 56,3 |
| 200 | 53,1 | 66,7 | 75,0 |
| 250 | 66,4 | 83,3 | 93,8 |
| 300 | 79,6 | 100,0 | 112,5 |
| 350 | 92,9 | 116,7 | 131,3 |

C'est à l'aide de modélisations du volume cellulaire de ce type de bactéries (Vc ; ul / mg de protéines bactériennes) qu'il est possible d'établir les mOSM nécessaires pour éviter de telles explosions cellulaires. Elles sont ici au minimum d'environ 200 mM pour les bactéries produites à des OSMc d'à peine 50, et d'environ 330 pour celles produites à des OSMc de 1,00 (Tableau 6). Ces molarités minimales impliquent qu'il faut non seulement fixer a priori le volume de la pré - bouillie (Tableau 4), mais aussi celui de la bouillie une fois le facteur de concentration (Figure 2) aboli. Il fallut calculer les volumes d'eau à ajouter aux pré - bouillies de manière à ne pas dépasser ces seuils de mOSM minimales (Tableaux 5a, 5b et 5c) fonction eux de l'OSMc (osmolarité de croissance) imposées aux bactéries leurs de leurs production (Tableau 6 ; *infra*), faute de quoi les bactéries seront en situation d'hypo - osmolarité et sujette à explosion (*i.e.* expansion exponentielle et très rapide de leurs Vc en fonction d'une diminution de OSMp (Figure 5).

L'osmo- tolérance des souches bactériennes ne sera qu'induite par ce passage transitoire au sein de la pré-bouillie. En ce sens, il est avantageux de faire appel à différents modes d'obtention de souches osmo- tolérantes, voire ici plus particulièrement du genre *Stenotrophomonas* (Bollet *et al.* 1995, Juhnke et Des Jardin 1989, ou encore Palleroni et Bradbury 1993) . Pour obtenir des souches osmotolérantes de familles taxonomiques choisies parmi un groupe comprenant les *Xanthomonadaceae* et les *Pseudomonadaceae,* il est utile d'impose une certaine pression osmotique environnement pendant leur croissance *in vitro* ; on parle alors encore une fois d'*osmolarité de croissance* (OSMc) . En effet, selon Cayley *et al.* (1991, 2000) des osmolarité allant de 50 à plus de 1 000 mOSM vont progressivement réduire le *volume cellulaire* (Vc ; uL/mg de protéine) des cellules bactériennes de manière à en augmenter leurs éventuelles osmo- tolérance. L'augmentation progressive de OSMc va aussi augmenter progressivement l'osmolarité minimale à maintenir dans la bouillie *in baco*, faute de quoi ces cellules osmotolérantes plus petites vont exploser en raison d'une augmentation soudaine de leur Vc en réponse à choc hypo- osmotique.

D'éventuelles *Stenotrophomonas* ne seront pas résistantes aux antibiotiques de types *imipenem* et/ou *beta-lactam,* ceux-ci pouvant produire des *imipenemases* et/ou surproduire des *beta-lactamases* notoires pour leur rôle dans certaines infections nosocomiales. Il est aussi utile de rechercher des *Stenotrophomonas* chitionolytiques, les *Stenotrophomonas* étant particulièrement abondantes au sein de tels contingents (Metcalfe *et al.* 2002a, 2002b).

Une fois la prébouillie constituée, un délai d'induction statique est respecté, soit ici de l'ordre de 8 à 12 heures à titre d'exemple. Le facteur de concentration appliqué lors de la constitution de ces prébouillies (Tableau 4) peut maintenant être aboli en apportant un volume d'eau (Tableaux 5a, 5b et 5c) de manière à ce que la mOSM de ces bouillies pulvérisables respectent les mOSM minima correspondant aux mOSM intracellulaires des bactéries produites à des mOSM de croissance (OSMc) plus ou moins importantes (Tableau 6). A noter enfin que ces mOSM minimales à respecter lors de la dilution de la prébouillie augmentent en fonction des susdits OSMc. L'*induction statique* de l'osmo-tolérance des BFCP, et plus particulièrement ici appartenant au genre *Stenotrophomomas,* peut être observé au sein d'une simple solution saline à un moment donné après induction statique au sens de la présente invention, soit avantageusement après une période de conditionnement au sein d'une pré-bouillie d'environs 500 mOSM de 10 à 12 heures, soit une période équivalente à une nuit dès la veille de la pulvérisation au champ par l'agriculteur.

**Tableau 5 a :** Volume d'eau à ajouter aux volumes des pré - bouillies indiquées au Tableau 4 en fonction de la dose hectare (Uha) du produit, du poids moléculaire des sels fertilisant constituant le constituant (g/mole), leur concentration dans le produit (g-sel/L) afin de respecter une mini-osmolarité (mOSM) minimale. A noter que cette mOSM minimale ($\alpha_2$ ; Tableau 6) est elle fonction de l'osmolarité de croissance (OSMc ; Tableau 6). Il s'agit ici d'un produit dont le poids moléculaire est des 113.

| Volume de la prébouillie | | | | Volume à ajouter si PM = 113 | | | | | | |
|---|---|---|---|---|---|---|---|---|---|---|
| | Poids Moléculaire (PM) | | | mOSM minimal à respecter (OSMp ; $\alpha_2$) ; Tableau 6 | | | | | | |
| **Uha = 5 g-sel/L** | **113** | **90** | **80** | **190** | **210** | **230** | **270** | **280** | **305** | **330** |
| 50 | 4 | | | 7 | 6 | 5 | 4 | 3 | 3 | 2 |
| 100 | 9 | | | 14 | 12 | 10 | 8 | 7 | 6 | 5 |
| 150 | 13 | | | 22 | 18 | 16 | 11 | 10 | 8 | 7 |
| 200 | 18 | | | 29 | 24 | 21 | 15 | 14 | 11 | 9 |
| 250 | 22 | | | 36 | 31 | 26 | 19 | 17 | 14 | 11 |
| 300 | 27 | | | 43 | 37 | 31 | 23 | 21 | 17 | 14 |
| 350 | 31 | | | 51 | 43 | 36 | 26 | 24 | 20 | 16 |
| | Poids Moléculaire (PM) | | | mOSM minimal à respecter (OSMp ; $\alpha_2$) ; Tableau 6 | | | | | | |
| **L/ha=10 g-sel/L** | **113** | **90** | **80** | **190** | **210** | **230** | **270** | **280** | **305** | **330** |
| 50 | 9 | | | 14 | 12 | 10 | 8 | 7 | 6 | 5 |
| 100 | 18 | | | 29 | 24 | 21 | 15 | 14 | 11 | 9 |
| 150 | 27 | | | 43 | 37 | 31 | 23 | 21 | 17 | 14 |
| 200 | 35 | | | 58 | 49 | 42 | 30 | 28 | 23 | 18 |
| 250 | 44 | | | 72 | 61 | 52 | 38 | 35 | 28 | 23 |
| 300 | 53 | | | 87 | 73 | 62 | 45 | 42 | 34 | 27 |
| 350 | 62 | | | 101 | 86 | 73 | 53 | 49 | 40 | 32 |
| | Poids Moléculaire (PM) | | | mOSM minimal à respecter (OSMp ; $\alpha_2$) ; Tableau 6 | | | | | | |
| **L/ha = 15 g-sel/L** | **113** | **90** | **80** | **190** | **210** | **230** | **270** | **280** | **305** | **330** |
| 50 | 13 | | | 22 | 18 | 16 | 11 | 10 | 8 | 7 |
| 100 | 27 | | | 43 | 37 | 31 | 23 | 21 | 17 | 14 |
| 150 | 40 | | | 65 | 55 | 47 | 34 | 31 | 25 | 21 |
| 200 | 53 | | | 87 | 73 | 62 | 45 | 42 | 34 | 27 |
| 250 | 66 | | | 108 | 92 | 78 | 57 | 52 | 42 | 34 |
| 300 | 80 | | | 130 | 110 | 93 | 68 | 63 | 51 | 41 |
| 350 | 93 | | | 152 | 128 | 109 | 79 | 73 | 59 | 48 |

**Tableau 5 b :** Volume d'eau à ajouter aux volumes des pré - bouillies indiquées au Tableau 4 en fonction de la dose hectare (Uha) du produit, du poids moléculaire des sels fertilisant constituant le constituant (g/mole), leur concentration dans le produit (g-sel/L) afin de respecter une mini-osmolarité (mOSM) minimale. A noter que cette mOSM minimale ($\alpha_2$ ; Tableau 6) est elle fonction de l'osmolarité de croissance (OSMc ; Tableau 6). Il s'agit ici d'un produit dont le poids moléculaire est des 90.

| Volume de la prébouillie | Volume à ajouter si PM = 90 |
|---|---|
| | |

(suite)

| Uha = 5 g-sel/L | Poids Moléculaire (PM) | | | mOSM minimal à respecter (OSMp ; $\alpha_2$) ; Tableau 6 | | | | | | |
|---|---|---|---|---|---|---|---|---|---|---|
| | 113 | 90 | 80 | 190 | 210 | 230 | 270 | 280 | 305 | 330 |
| 50 | | 6 | | 9 | 8 | 7 | 5 | 4 | 4 | 3 |
| 100 | | 11 | | 18 | 15 | 13 | 9 | 9 | 7 | 6 |
| 150 | | 17 | | 27 | 23 | 20 | 14 | 13 | 11 | 9 |
| 200 | | 22 | | 36 | 31 | 26 | 19 | 17 | 14 | 11 |
| 250 | | 28 | | 45 | 38 | 33 | 24 | 22 | 18 | 14 |
| 300 | | 33 | | 54 | 46 | 39 | 28 | 26 | 21 | 17 |
| 350 | | 39 | | 63 | 54 | 46 | 33 | 31 | 25 | 20 |

| L/ha=10 g-sel/L | Poids Moléculaire (PM) | | | mOSM minimal à respecter (OSMp ; $\alpha_2$) ; Tableau 6 | | | | | | |
|---|---|---|---|---|---|---|---|---|---|---|
| | 113 | 90 | 80 | 190 | 210 | 230 | 270 | 280 | 305 | 330 |
| 50 | | 11 | | 18 | 15 | 13 | 9 | 9 | 7 | 6 |
| 100 | | 22 | | 36 | 31 | 26 | 19 | 17 | 14 | 11 |
| 150 | | 33 | | 54 | 46 | 39 | 28 | 26 | 21 | 17 |
| 200 | | 44 | | 73 | 61 | 52 | 38 | 35 | 28 | 23 |
| 250 | | 56 | | 91 | 77 | 65 | 47 | 44 | 36 | 29 |
| 300 | | 67 | | 109 | 92 | 78 | 57 | 52 | 43 | 34 |
| 350 | | 78 | | 127 | 107 | 91 | 66 | 61 | 50 | 40 |

| L/ha=15 g-sel/L | Poids Moléculaire (PM) | | | mOSM minimal à respecter (OSMp ; $\alpha_2$) ; Tableau 6 | | | | | | |
|---|---|---|---|---|---|---|---|---|---|---|
| | 113 | 90 | 80 | 190 | 210 | 230 | 270 | 280 | 305 | 330 |
| 50 | | 17 | | 27 | 23 | 20 | 14 | 13 | 11 | 9 |
| 100 | | 33 | | 54 | 46 | 39 | 28 | 26 | 21 | 17 |
| 150 | | 50 | | 82 | 69 | 59 | 43 | 39 | 32 | 26 |
| 200 | | 67 | | 109 | 92 | 78 | 57 | 52 | 43 | 34 |
| 250 | | 83 | | 136 | 115 | 98 | 71 | 65 | 53 | 43 |
| 300 | | 100 | | 163 | 138 | 117 | 85 | 79 | 64 | 52 |
| 350 | | 117 | | 190 | 161 | 137 | 99 | 92 | 75 | 60 |

**Tableau 5 c :** Volume d'eau à ajouter aux volumes des pré - bouillies indiquées au Tableau 4 en fonction de la dose hectare (Uha) du produit, du poids moléculaire des sels fertilisant constituant le constituant (g/mole), leur concentration dans le produit (g-sel/L) afin de respecter une mini-osmolarité (mOSM) minimale. A noter que cette mOSM minimale ($\alpha_2$ ; Tableau 6) est elle fonction de l'osmolarité de croissance (OSMc ; Tableau 6). Il s'agit ici d'un produit dont le poids moléculaire est des 80.

| Volume de la prébouillie | Volume à ajouter si PM = 80 |
|---|---|

| Uha = 5 g-sel/L | Poids Moléculaire (PM) | | | mOSM minimal à respecter (OSMp ; $\alpha_2$) ; Tableau 6 | | | | | | |
|---|---|---|---|---|---|---|---|---|---|---|
| | 113 | 90 | 80 | 190 | 210 | 230 | 270 | 280 | 305 | 330 |
| 50 | | | 6 | 10 | 9 | 7 | 5 | 5 | 4 | 3 |
| 100 | | | 13 | 20 | 17 | 15 | 11 | 10 | 8 | 6 |
| 150 | | | 19 | 31 | 26 | 22 | 16 | 15 | 12 | 10 |
| 200 | | | 25 | 41 | 35 | 29 | 21 | 20 | 16 | 13 |
| 250 | | | 31 | 51 | 43 | 37 | 27 | 25 | 20 | 16 |
| 300 | | | 38 | 61 | 52 | 44 | 32 | 29 | 24 | 19 |

(suite)

| Uha = 5 | Poids Moléculaire (PM) | | | mOSM minimal à respecter (OSMp ; $\alpha_2$) ; Tableau 6 | | | | | | |
|---|---|---|---|---|---|---|---|---|---|---|
| | 113 | 90 | 80 | 190 | 210 | 230 | 270 | 280 | 305 | 330 |
| 350 | | | 44 | 71 | 60 | 51 | 37 | 34 | 28 | 23 |

| L/ha=10 | Poids Moléculaire (PM) | | | mOSM minimal à respecter (OSMp ; $\alpha_2$); Tableau 6 | | | | | | |
|---|---|---|---|---|---|---|---|---|---|---|
| | 113 | 90 | 80 | 190 | 210 | 230 | 270 | 280 | 305 | 330 |
| g-sel/L | | | | | | | | | | |
| 50 | | | 13 | 20 | 17 | 15 | 11 | 10 | 8 | 6 |
| 100 | | | 25 | 41 | 35 | 29 | 21 | 20 | 16 | 13 |
| 150 | | | 38 | 61 | 52 | 44 | 32 | 29 | 24 | 19 |
| 200 | | | 50 | 82 | 69 | 59 | 43 | 39 | 32 | 26 |
| 250 | | | 63 | 102 | 86 | 73 | 53 | 49 | 40 | 32 |
| 300 | | | 75 | 122 | 104 | 88 | 64 | 59 | 48 | 39 |
| 350 | | | 88 | 143 | 121 | 103 | 75 | 69 | 56 | 45 |

| L/ha=15 | Poids Moléculaire (PM) | | | mOSM minimal à respecter (OSMp ; $\alpha_2$) Tableau 6 | | | | | | |
|---|---|---|---|---|---|---|---|---|---|---|
| | 113 | 90 | 80 | 190 | 210 | 230 | 270 | 280 | 305 | 330 |
| g-sel/L | | | | | | | | | | |
| 50 | | | 19 | 31 | 26 | 22 | 16 | 15 | 12 | 10 |
| 100 | | | 38 | 61 | 52 | 44 | 32 | 29 | 24 | 19 |
| 150 | | | 56 | 92 | 78 | 66 | 48 | 44 | 36 | 29 |
| 200 | | | 75 | 122 | 104 | 88 | 64 | 59 | 48 | 39 |
| 250 | | | 94 | 153 | 129 | 110 | 80 | 74 | 60 | 48 |
| 300 | | | 113 | 184 | 155 | 132 | 96 | 88 | 72 | 58 |
| 350 | | | 131 | 214 | 181 | 154 | 112 | 103 | 84 | 68 |

## APPLICATION BIOINDUSTRIELLE ET AGRONOMIQUE

*Production des biomasses bactériennes a priori osmotolérantes*

La production de biomasses bactériennes a *priori* osmotolérantes est bien connu de l'homme du métier. L'approche la plus simple est d'appliquer (de réaliser) une osmolarité appréciable au cours de la production par fermentation, liquide ou solide, desdites population bactériennes. Les bactéries ainsi produites en masse seront nécessairement plus résistantes, non seulement aux stresses osmotiques, mais aussi parfois aussi aux stresses thermiques (Besten *et al*. 2006), voire oxydatifs, acido-basiques, *etc*. (Bonaterra *et al*. 2007) Une des caractéristique des bactéries osmotolérantes ainsi isolées et produites en masses en présence d'osmolarités grandissantes est une réduction de leurs volumes cellulaire, Vc, et du coup de leur encombrement moléculaire (Cayley *et al*. 2000, Zimmerman et Trach 1991, Ellis 2001, Spitzer 2011, Spitzer et Poolman 2005) La mesure de cette osmotolérance et de ces caractéristiques morphocellulaire telles que le susdit encombrement moléculaire est connue de l'homme du métier.

Les notions *d'osmolarité de croissance* (OSMc) et *d'osmolarité de plasmolyse* (OSMp) évoquées plus bas préciseront d'autant plus les conditions de culture et de production des BFCP a priori osmotolérantes. En effet, c'est l'osmolarité vécue par les bactéries pendant leur croissance (OSM de croissance ; OSMc au sens de Cayley *et al*. 2000) qui détermine leurs omolarité de plasmolyse (OSMp ; *op. cit*.). Or, cette OSMp reflète précisément le degré d'osmotolérance ad'une bactérie lui permettant de contracter (concentrer) au maximum son volume cellulaire (Vc ; uL / mg-MS) avant d'imploser (plasmolyse ; *op. cit.,* figure 1). Ce lien entre OSMc et OSMp permet donc de connaître a *priori* le degré d'osmotolérance des bactéries ainsi produites, et du coup d'ajuster l'osmolarité de la pré - bouillie en conséquence (voir *infra*).

A ce stade de développement de l'invention il est loisible d'utiliser les valeurs paramétriques applicables à une simple bactérie Gram⁻ telle qu'*E. coli*; elles pourront toujours être précisées spécifiquement pour diverses espèces bactériennes le moment venu par titrage NaCl au sens de Cayley *et al*. 2000, ou encore plus simplement Pilizota et Shaevitz 2012. Voir aussi une revue de certaines de ces méthodes de titrage pour la détermination de Vc en annexe de Wood 1999 (*op. cit.* pp. 254-255).

Cela dit, c'est surtout ici OSMc qui dictera la valeurs de OSMp ($\alpha_2$ ; Tableau 6), et rien ne laisse croire à ce stade les coefficients (variables paramétriques) de courbes telles que celles ici à la Figure 6 mais pour des espèces autres qu'*E. coli* donneraient des OSMp appréciablement différents des ceux rapportés ici au Tableau 6. En effet et par exemple, les osmolarités imposées à des *Pseudomonas syringae* variant ici de 128 à 385 mOSM (Tableaux 1 et 2) sont très comparables à celles imposées à *E. coli qui* elles varient de 182 à 463, et du coup parfaitement dans la moyenne (430 mM ; Tableau 1). De plus, la valeur de l'activité de l'eau ($a_w$) limitant la croissance d'une bactérie est essentiellement la même (~0,95) pour *E. coli, Pseudomonas* et *Salmonella,* voire la plupart des bactéries Gram[-], soit entre 0,94 et 0,96 (par opposition à ~0,80 pour les champignons par exemple).

Une fois produite en masse, les bactéries peuvent être formulées et conditionnées selon l'état de l'art, soit sous forme solide, voir par lyophilisation, soit en formulation liquide en présence de PVPP, CMC, glycerol, etc.

*Pré-application des BFCP (« pré-bouillie »)*

L'utilisateur peut réaliser la pré - bouillie permettant *l'induction statique* des bactéries osmotolérantes en remplissant que partiellement la cuve (*in baco*) du pulvérisateur, laisser « incuber » pendant 12 heures, soit usuellement depuis la veille, et compléter par la suite le volume de la bouillie au moment ou peu avant l'application de la préparation au champ, et cela en fonction des volumes préconisés ici aux Tableau 5-a, b et c. Selon la concentration (g/L) des produites fertilisants appliqués conjointement, le poids moléculaire (g/mole) de leurs principales composantes, et le volume de la bouillie préconisé, les volumes de la pré - bouillie sont indiquées au Tableau 4. Expérimentalement, nous n'aurons qu'à simuler la formation de cette pré - bouillie concentrée par un facteur (de concentration) donnée à l'aide de solutions.

*Application in situ des BFCP*

Les préparations bactériennes au sens de la présente invention peuvent avantageusement être appliqués aux feuillages, et cela conjointement à certains agents azoto-nutritionnels (ANN), voire à certains fongicides, régulateurs de croissance et/ou toutes autres matières fertilisantes et/ou phytosanitaires applicables aux feuillages. Plus particulièrement il pourrait ici être question d'engrais foliaires types Azofol™, Fertigofol™ et AzoSpeed™ commercialisés par Agronutrition SAS (France). Il peut aussi être question d'ANN tel que Optéine™, Phyléas™, voir plus avantageusement au sens de FR0900867/EP10366001.5 ; cette dernière technologie permet en effet une « fenêtre d'application » beaucoup plus large et donc plus opportune.

*Modélisations des Vc,mini et des osmolarité minimale à respecter*

Modélisons l'effet de la création des pré - bouillies, notamment ici au sens de Cayley *et al.* 2000 (*op. cit.,* figures 1 et 6), et Dötsch *et al.* 2008 (*op. cit.,* éq. 7). Il faut aussi tenir compte des notions d'enzymes bactériennes halophiles et halotolérantes (Sleator et Hill 2001). En effet, il existe vraisemblablement un gradient entre une *halophilie* stricte nécessitant de très fortes osmolarités à hauteurs de 5 à 7 molaire (Wood 1999, tableau 1), et une certaine *halotolérance* en présence d'osmolarité d'au plus équivalentes à 0,5 molaire. Les bactéries au sens de la présente invention sont avantageusement intermédiaires ; leurs enzymes pouvant bénéficier de la stabilisation que procure des cations à forte concentration - un peu à la façon des bactéries halophiles, tout en accumulant des osmolytes comme le font les bactéries halotolérantes.

Selon l'équation apparaissant dans Dötsch *et al.* 2008 ;

$$V_c = \alpha_1 / (OSMp + \alpha_2) + V_{c,min} \qquad (1)$$

$V_c$ = volume cellulaire aqueux (uL / mg-matière sèche (MS))
$\alpha_1$ = paramètre empirique (ul / mg-protéine)
OSMp = milli - osmolarité de plasmolyse (via titrage NaCl ; Cayley *et al.* 2000)
$\alpha_2$ = paramètre empirique (mol / L)
$V_{c,min}$ = volume cellulaire aqueuse minia (uL / mg-MS)

... et à partir de données numériques telles que celles présentées par Cayley *et al.* 2000 pour *E. coli* (op. cit. tableau 1), il est maintenant possible de déterminer les paramètres $\alpha_1$, $\alpha_2$ et $V_{c,min}$ pour une gamme de valeurs mOSM de croissance (OSMc). Il est donc possible de recalculer les valeurs d'osmolarité minimales à prévoir dans la pré - bouillie afin d'éviter l'explosion des BFCP.

Cette modélisation peut s'effectuer via un algorithme stochastique compris dans une pack statistique aussi banale que XLStat™; voir en ce sens les valeurs paramétriques au Tableau 6. Cette modélisation de Vc selon OSMp et OSMc est représentée à la Figure 4. Non seulement les bactéries les plus osmotolérantes ont les Vc les plus réduits (i.e. une compression favorisant un plus grand encombrement moléculaire propice à une activité métabolique plus soutenue à omolarités élevées), mais elles nécessitent aussi des osmolarités minimales plus élevées que des bactéries moins osmotolérantes produite à des osmolarité de croissance (OSMc) plus faibles, faute de quoi les Vc augmenteront très rapidement, les cellules bactériennes devant ainsi nécessairement exploser si l'osmolarité ambiante n'est pas suffisante (i.e. d'au moins 190 à 330 mOSM, selon le cas). Des bactéries croissant en présence d'OSMc d'environs 0,5 à 0,8 molaire devront impérativement être mises en présence d'au moins 250 à 300 mOSM.

A noter ici que la variable $\alpha_2$ mesure approximativement de la molarité minimale à respecter dans la pré - bouille afin d'éviter l'explosion des BFCP plus ou moins osmotolérante selon leur OSMc. En effet, la valeur paramétrique de cette variable coïncide avec le point d'inflexion de la susdite équation non - linéaire rationnelle. Une population de BFCP produite à un OSMc donnée placée dans une une pré - bouillie dont l'osmolarité n'est pas d'au moins $\alpha_2$ explosera à plus ou moins brève échéance, vraisemblablement avant d'avoir été pulvérisée au feuillage. A noter aussi que les valeurs $\alpha_2$ sont ici, pour des OSMc correspondant aux alentours de 0,500 (de 0,300 à 0,600 ; Tableau 6), d'environ la moitié de la milli - osmolarité en principe la plus avantageuse préconisée pour la pré - bouillie, i.e. approximativement 500 milli - molaire. Or, selon la Figure 5, des bactéries produites à de telles OMSc peuvent résister à des OSMp bien au delà de 0,500. C'est pourquoi nous avons proposé pour les pré - bouillie une gamme de milli-osmolarités (mOSM) comprise entre 250 et 1 250 milli - molaire.

**Tableau 6 :** Valeurs des variables paramétriques $\alpha_1$, $\alpha_2$ et $V_{c,min}$ pour le calcul de l'osmolarité de plasmolyses (OSMp) de la pré - bouillie selon l'osmolarité de croissance (OSMc) lors de la production des bactéries. (cf. représentation graphique, ici Figure 6).

| Osmolarité croissance (OSM-c) | $\alpha_1$ | Osmolarité de plasmolyse (OSMp ; $\alpha_2$) | Volumes cellulaires minima ($V_{c,mini}$) |
|---|---|---|---|
| *mol/L* | *uL / mg-protéine* | *mol/L* | *uL / mg-protéine* |
| 0,05 | 0,241 | 0,190 | 1,858 |
| 0,10 | 0,260 | 0,210 | 1,800 |
| **0,30** | **0,350** | **0,230** | **1,680** |
| **0,60** | **0,500** | **0,270** | **1,530** |
| 0,70 | 0,550 | 0,280 | 1,480 |
| 0,90 | 0,650 | 0,305 | 1,384 |
| 1,00 | 0,740 | 0,330 | 1,260 |

Concrètement, afin de compléter le Tableau 6, j'ai extrapoler linéairement à entre les osmolarité de croissance (OSMc) de 0, 03 et 0, 83 osmolaire (OSM) lors de la production des biomasses d'*E. coli*- une banale bactérie Gram-négative. A noter la différence ici entre les valeurs d'OSMp obtenues par *titrage- NaCl* (Cayley *et al*. 2000), et d'OSMc imposées lors des cultures par fermentation liquide desdites *E. coli* ; c'est donc ici OSMc qui dicte OSMp, et donc le niveau d'osmotolérance a *priori* de ces bactéries. De plus, *E. coli* n'est pas en soi une bactérie tellurique et/ou particulièrement robuste. L'application à des bactéries du sols et/ou de la phyllosphère des paramètres ainsi recalculer à partir de ceux d'*E. coli* est donc une approche conservatrice.

J'ai représenté graphiquement à la Figure 6 une telle progression quasi linéaire des osmolarités minima des pré - bouillie inférées à partir de OSMp ($\alpha_2$ ; éq. (1)) pour diverses OSMc. Sans être universelle, cette courbe est a *priori* et à ce stade applicable à la plupart des BFCP Gram⁻, l'activité de l'eau ($a_w$) limitant la croissance étant essentiellement la même (~0,95) pour des bactéries telles que *E. coli, Pseudomonas* et *Salmonella,* voire la plupart des bactéries Gram⁻. Cela dit, et à ce stade qu'approximativement, les progressions quasi linéaire des OSMp minima pour des bactéries Gram⁺, en principe plus halophiles les Gram⁻, seront elles décrites par une fonction dont la courbe (pointillée) est légèrement mais nécessairement déplacée vers le haut (+10% ; pointillé Figure 6). *Idem* pour ce qui concernerait d'éventuelles bactéries osmotolérantes moins halophiles, les osmolarités minima de la bouillie serait elles décrites par une courbe (pointillée) légèrement mais nécessairement déplacée vers le bas (-10% ; pointillé Figure 6). En effet, cette relative suraccumulation d'osmolytes minéraux et organiques par des bactéries plutôt halophiles telle que Halomonas elongata DSM 2581 est clairement décrit par Dötsch *et al*. 2008. Des titrages - NaCl tel que décrits dans Cayley *et al*. 2000 spécifique pourront aussi être effectués pour diverses espèces de BFCP pour confirmer cette applicabilité interspécifique des courbes à la Figure 6.

A l'aide de ces *régressions non - linéaires rationnelles* (Cayley *et al*. 2000, figure 1) de modéliser le volume cellulaire aqueux ($V_c$) en fonction d'OSMp. Du coup, via la valeur paramétrique $\alpha_2$ nous pouvons aussi estimer la valeur minimale de l'osmolarité en dessous des quelle les BFCP produites à un OSMc particulière risquent d'exploser, $V_c$ tendrant rapidement vers des valeurs extrêmes au delà de 3,0 ul / mg-MS. Ces valeurs minimales de $V_c$ révèlent l'importance de maintenir la molarité de la pré - bouillie à au moins 0,200, voire plus de 0,300 osmolaire. Il est ainsi possible d'assurer l'induction de l'osmotolérance des BFCP, mais aussi la survie dans les bouillies pulvérisables.

Par exemple, une population bactérienne produite à une mOSMc de 50 devra être toujours en présence d'une mOSMp d'au moins 190 une fois la bouillie pulvérisable restaurée après abolition du facteur de concentration. En ce sens, pour un produit de poids moléculaire (PM) de 80, une concentration du produit de 250 g/L au sein d'une pré - bouillie de 51 L installée pendant 12 heures et préconisant 5 L/ha de produit, il ne faudra pas ajouter plus de 31 L d'eau à la pré - bouillie, créant ainsi une bouillie pulvérisable d'au plus 51 + 31 = 52 L/ha.

*Validation via PV = nRT*

L'invention est applicable à toutes les biomasses bactériennes expressément osmotolérantes parce que produites en présence de pression osmogènes de culte (OSMc) de l'ordre de 0, 05 à 1, 00 mol/ L ; en ce sens ces souches ne sont pas nécessairement halophiles mais plus simplement osmotolérantes. Les volumes qu'occuperont les cytosols de ces microorganismes à diverses OSMc et OSMp (osmolarité de plasmolyses ; Tableau 6) sont fonction du volume *osmoactif* ($V_{osmoactif}$ ; ul/mg- protéine microbienne) de ces cellules, en non du volume *non- osmoactif* ($V_{nonosmoactif}$) . Or, en raison de l'équation empirique $[V_c = \alpha_1 / (OSMp + \alpha_2) + V_{c,min,}]$ applicable au volume cellulaires bactériens (*supra,* equation (1) ), ces volumes peuvent être exprimés en termes de pressions, et cela grossièrement en accord avec la loi universelle régissant le volume d'un gaz (parfait), soit ;

$$pV = nRT$$

- $p$ est la pression du gaz (pascal) ;
- $V$ est le volume occupé par le gaz (mètre cube) ;
- $n$ est la quantité de matière (mole) ; ne pas confondre avec $N$ est le nombre de particules ;
- $R$ est la constante universelle des gaz parfaits : $R = 8, 314\ 472\ J \cdot K^{-1} \cdot mol^{-1}$
- $T$ est la température absolue (en kelvin) ;

De plus, selon Klipp *et al*. 2005 et Cayley *et al*. 1991, 2000 ;

$$V_c = V_{nonosmactif} + V_{osmoactif}$$

$$\pi_{turgescence} = \pi_{interne} - \pi_{externe}$$

C'est comme de raison le volume osmoactif ($V_{osmoactif}$), i.e. le volume cellulaire affecté par la pression osmotique externe ($\pi_e$), qui dictera ainsi quel pression cellulaire interne ($\pi_i$) il faut maintenir pour assurer la turgescence de la bactérie. Il est donc ainsi possible de démontrer que la pression *inter*cellulaire ($\pi_i$) est égale à ;

$$RT \times (n/_{V_{osmoactif}})$$

De même, il est aussi possible de démontrer que la pression extracellulaire ($\pi_e$) à maintenir pour éviter une perte de turgescence cellulaire (bactérienne) est elle égale à ;

$$RT \times (n/V_{bouillie})$$

Le volume de la bouillie, $V_{bouillie}$, est en effet celui de la solution *minérale in baco* extérieur à cellule microbienne tel que prescrit à titre d'exemple de mode de réalisation *supra* aux Tableaux 4, 5a, 5b et 5c. En effet, les volumes des pré - bouillies (Tableau 4) et les volumes d'eau à leur ajouter afin d'obtenir un volume de bouillie respectant OSMp (Tableaux 5a, 5b et 5c) peuvent ainsi être directement obtenus pour une gamme de produits minéraux fertilisants, et cela ici afin d'assurer une mOSM d'environ 500 favorable à l'induction de l'osmotolérance de bactéries moyennement halophiles (cf. Tableaux 1 et 2). Comme de raison, il est aussi loisible de compléter ces tableaux pour d'autre produits aux poids moléculaires (PM) et concentrations différents.

Toujours est-il que pour que la turgescence des cellules microbiennes ne soit pas affectée par leur immersion dans le bac du pulvérisateur contenant les sels fertilisants, il faut donc que $\pi_{interne} = \pi_{externe}$ ($\pi_i = \pi_e$), ce qui amène l'équation suivante ;

$$nRT / V_{osmoactif} = nRT / V_{bouilllie}$$

Donc, pour valider que le $V_{osmoactif}$ fonction de OSMc (Tableau 6, Figure 6), et donc la turgescence des cellules microbienne sont effectivement préservés, il suffit de s'assurer que $V_{osmoactif}$ et $V_{bouille}$ sont proportionnels, voire dans une relation quasi linéairement (Figure 7) . A noter que pour ce faire, il est important d'exprimer $V_{bouille}$ non pas en L/ha, mais bien en L/kg de sel fertilisant de manière à rendre $V_{bouillie}$ directement comparable à $V_{osmoactif}$ exprimé lui aussi pondéralement (uL/mg de protéine protéine) . Les unités de $V_{osmoactif}$ et $V_{bouillie}$ sont ainsi commensurables ; [uL, L : mg, Kg] . Une fois cette transformation des $V_{bouille}$ repérés aux Figures 4 et 5 effectuée, cette proportionnalité des $V_{osmoactif}$ et $V_{bouillie}$ est reproduite graphiquement à la Figure 7 ; à noter qu'elle ne dépends pas de la concentration et ou des dose- hectare des produits, mais seulement de leurs poids moléculaire (PM ; g/mol), soit ici et à titre d'exemple de 80 à 113.

Selon la Figure 7, le pouvoir osmogène des sels est de 40 à 60 fois, selon le PM du sel fertilisant, plus important que celui des protéines bactériennes. Or, cela est cohérent avec une PM *moyen* aux alentours de 5 000 (5 kDa) pour l'*ensemble* des protéines et/ou peptides bactériens. En effet, bien que le poids usuel d'une protéine bactérienne est plus élevé, de l'ordre de 10 à 12 kDa, cela ne comprend que les protéines complètement assemblées. Le cytosol bactérien est en effet composés non seulement de telles protéines *fonctionnelles,* mais aussi et surtout de leurs parties nécessairement plus petites.

*Impacts agronomiques escomptés (voir aussi data Bonaterrea et al. 2007)*

L'application agronomique de l'invention permettra d'induire en temps utile l'osmotolérance des BFCP expressément isolées et produites à cet effet, et cela tout en préservant l'intégrité des cellules bactériennes ainsi plus osmotolérantes au moment de leur application *in situ (in folio).* En effet, l'osmotolérance bactérienne est souvent assez transitoire, ou moins non constitutive ; la valorisation agronomique est donc souvent problématique en raison d'un décalage entre le moment d'application foliaire de tels inoculum et l'activation métabolique de la cellule, y compris de son osmotolérance. De plus, cette osmotolérance est particulièrement requise si ladite application foliaire ce fait conjointement à celle de sels fertilisants ; l'évaporation de l'eau à la surface du feuillage va ainsi très rapidement concentrer les sels provoquant une importante pression osmotique subie par les bactéries.

Ce type de BFCP *a priori* osmotolérantes, voire encore plus avantageusement chitinolytiques, et maintenant induites statiquement seront d'autant plus efficaces entant qu'agents fongistatiques, *stomatoactifs* capables par exemple d'augmenter la conductivité *stomatique* ($g_s$ ; mole/m$^2$/sec).

A titre illustratif à ce stade, voir ici la Figure 2 résumant graphiquement les données de Bonaterra *et al*. 2005 et 2007. Les deux expérimentations concernent des bactéries osmo - adaptées à 0,5 $M_{NACl}$ (figure du haut ; data Bonaterra 2005) et 0,7 $M_{NaCl}$ (figure du bas ; data Bonaterra 2007). A titre illustratif j'ai donc placé une courbe (en pointillé) escomptant la survie de telles bactéries dont l'osmolarité de la suspension (bouillie) aurait été ajusté en fonction de leurs l'osmolarités de croissance (OSMc) au sens de la présente invention. A noter enfin que ces cinétiques décrivent la mortalité bactérienne due à la dessiccation *in vitro ;* celle ci est parfaitement corrélée à la survie *in vivo (in folio)* de telles bactéries (*eg*. Bonaterra *et al*. 2007, figs. 3b et 3e ) et sert donc souvent ainsi de mesure de substitution.

## SIGLES ET DEFINITIONS

| | | | |
|---|---|---|---|
| **BFCP** : | bactéries favorisant la croissance des plantes | **mOSM** : | milli-osmolarité |
| **SDN** : | stimulation des défenses naturelles (de la plante) | **OSMc** : | osmolarité ce croissance |
| **GG** : | glucosyl-glycérol | **OSMp** : | osmolarité de plasmolyse |
| **GGate** : | glucosyl-glycérate | **Vc (Vc,min)** : | volume cellulaire (aqueux), minimum |
| **GgpS** : | GG phosphate synthase | **V$_{bouillie}$ :** | volume (L) de la bouillie pulverisable |
| **GgpP** : | GG phosphate phosphatase | **V$_{osmoactif}$** : | volume osmoactif du cytosol bactérien |

(suite)

| NAGGN : | *N*-acetylglutaminylglutamine amide | V<sub>nonosmoactif</sub> : | volume non-osmoactif du cytosol bactérien |

**NAGGN** :   *N*-acetylglutaminylglutamine amide    $V_{nonosmoactif}$ :   volume non-osmoactif du cytosol bactérien

**CMC** :   carboxy-methyl-cellulose

**Facteur de concentration** : inverse du rapport entre la (milli- ) osmolarité (mOSM) de la bouillie pulvérisable et le seuil critique osmolaire préétabli induisant la production et/ou la suraccumulation d'osmolytes compatibles. Il permet d'établir de facto le volume de la pré- bouillie.

**Pré-bouillie** : mélange de sels fertilisants osmogènes, et éventuellement d'inocula dans un volume d'eau inférieur par un certain facteur de concentration à celui de la bouillie pulvérisable.

**Bouillie pulvérisable** : mélange de sels osmogènes dans un volume d'eau préconisé pour leur application foliaire.
*in* baco : directement dans le bac du pulvérisateur (*cf. in vitro, in situ, in planta, in silico, in saco, etc.*)
*Induction statique :* mécanisme d'adaptation des cellules bactériennes à des stresses - osmotique en particulier, par simple exposition, sans croissance des populations. En principe l'induction statique est à opposée à *l'induction dynamique* à de tels stresses suite à la croissance des population en milieux de culture enrichis.

**(milli- ) Osmolarité** (mOSM) : somme théorique des pressions osmotiques attribuable à l'ensemble des molécules ioniques et/ou ou osmotiquement actives dans une solution. A ne pas confondre avec l'osmolaité, *i.e.* la pression osmotique de ladite solution telle que mesurée, et ne pouvant être calculée.

**Osmola(r)ité de croissance / de plasmolyse :** l'osmolarité des milieux de culture lors de la croissance des bactéries est dite osmolarité de croissance (OSMc). Celle-ci dictera l'osmolarité de plasmolyse (OSMp), mesure obtenue par titrage - NaCl (Cayley *et al.* 2000) indiquant le niveau d'osmo-tolérance d'une bactérie ainsi capable de réduire au maximum son volume cellulaire avant d'imploser définitivement.

**Osmotolérance :** état induit d'un (micro) organisme, une bactérie non sporulante par exemple, lui permettant survivre, voire activement, à un stress osmotique non bactéricide bien que potentiellement bactériostatique.

**Halophilie :** état constitutif d'un (micro) organisme, une bactérie non sporulante par exemple, lui permettant survivre, voire activement, à un stress osmotique non bactéricide bien que potentiellement bactériostatique.

**Osmolyte compatible :** molécule osmotiquement actives mais non ou peu toxique pour la cellule microbienne ou végétale prélevée par celle-ci en réponse à une pression osmotique de façon à permettre l'exclusion d'osmolytes plus toxiques - tel que sodium ou le chlore, tout en maintenant une pression intracellulaire viable.

**Phyllosphère :** surface et tissus histologiques superficiels des feuilles (feuillage) ; ne pas confondre avec phylloplan désignant que la surface des feuilles.

**REFERENCES**

Bollet, C., A. Davin-Regli et P. De Micco. 1995. A Simple Method for Selective Isolation of Stenotrophomonas maltophilia from Environmental Samples. Appl. Environ. Microbiol. 61: 1653-54

Bonaterra, A, J Camps et E Montesinos. 2005. Osmotically induced trehalose and glycine betaine accumulation improves tolerance to desiccation, survival and e□cacy of the postharvest biocontrol agent Pantoea agglomerans EPS125. FEMS Microbiol. Lett. 250: 1-8

Bonaterra, A., J. Cabrefiga, J. Camps et E. Montesinos. 2007. Increasing survfival and efficacy of bacterial biocontrol agent of fire bligth of rosaceaous plants by meands of osmoadaptation. FEMS Microbiol. Ecol. 61 : 185-195.

Cayley, DS., HJ. Guttman et MT. Record, Jr. 2000. Biophysical characterization of changes in amounts and activity of E. coli cell and compartment water and turgor pressure in response to osmotic stress. Biophycisal. J. 78 : 1748-1764.

Cayley, DS, BA. Lewis, HJ. Guttman et MT. Record, Jr. 1991. Characterization of the cytoplasm of Escherichia coli K-12 as a function of external osmolarity : implications for protein - DNA interactions in vivo. J. Mol. Biol. 222 : 281-300.

Crossman, LC., VC Gould, JM Dow, GS Vernikos, a Okazaki, M Sebaihia, D Saunders, C Arrowsmith, T Carver, NP, E Adlem, a Kerhornou, a Lord, L Murphy, K Seeger, R Squares, S Rutter, MA Quail, M-A Rajandream, D Harris, C Churcher, SD Bentley, J Parkhill, NR Thomson and MB Avison. 2008. The Complete Genome, Comparative and Functional Analysis of Stenotrophomonas maltophilia Reveals an Organism Heavily Shielded by Drug Resistance Determinants. Genome Biology 2008, 9:R74.

Csonka, L.N. 1989. Physiological and genetic responses of bacteria to osmotic stress. Microbiol. Rev. 53:121-147.

Dôtsch, A., J. Severin, W. Alt, EA. Galinski et J-U. Kreft. 2008. A mathematical model for growth and osmoregulation in halophilic bacteria. Microbiology 154 : 2956-2969

Ellis, RJ. 2001. Macromolecular crowding : an important but neglected aspect of the intracellular environment. Curr.

Op. Struct. Biol. 11:114-119.

Ferjani, A., L. Mustardy, R. Sulpice, K. Marin, I. Suzuki, M. Hagemann et N. Murata. 2003. Glucosylglycerol, a Compatible Solute, Sustains Cell Division Under Salt Stress. Plant Physiol. 131 : 1628-1637

Giesler, LJ. et GY. Yuen. 1998. Evaluation of Stenotrophomonas maltophilia Strain C3 for Biocontrol of Brown Patch Disease. Crop Protect. 17:509-513

Goldman. S., K. Hecht, H. Eisenberg et M. Mevarech. 1990. Extracellular Ca2+-Dependent Inducible Alkaline Phosphatase from the Extremely Halophilic Archaebacterium Haloarcula marismortui. J. Bacteriol. 172 : 7065-70

Goude, R. S. Renaud, S. Bonnassie, T. Bernard et C. Blanco. 2004. Glutamine, Glutamate, and□-Glucosylglycerate are the Major Osmotic Solutes Accumulated by Erwinia chrysanthemi Strain 3937. Appl. Environ. Microbiol. 70 : 6535-6541

Gouesbet, G, A Trautwetter, S Bonnassie, LF Wu et C Blanco. 1996. Characterization of the Erwinia chrysanthemi osmoprotectant transporter gene ousA. J. Bacteriol. 78 : 447-455.

Hagemann, M., K. Ribbeck-Busch, S. Klähn, D. Hasse, R. Steinbruch et G. Berg. 2008. The Plant-Associated Bacterium Stenotrophomonas Rhizophila Expresses a New Enzyme for the Synthesis of the Compatible Solute Glucosylglycerol. J. Bacteriol. 190 : 5898-5906

Hagemann, M. et N. Erdmann. 1994. Activation and Pathway of glucosyl glycerol Synthesis in the Cyanobacterium Synechocystis Sp. Pcc 6803. Microbiology 140, 1427-1431

Heylen, K., B. Vanparys, F. Peirsegaele, L. Lebbe et P. De Vos. 2007. Stenotrophomonas Terrae Sp. Nov. and Stenotrophomonas Humi Sp. Nov., Two Nitrate-Reducing Bacteria Isolated from Soil. Intl. J. Syst. Evol. Microbiol. 57 : 2056-2061

Johan H., J. Leveau and Steven E. Lindow. 2001. Appetite of An Epiphyte: Quantitative Monitoring of Bacterial Sugar Consumption In The Phyllosphere. Pnas, Vol. 98 No. 6, P. 3446-3453

Juhnke, ME et E Des Jardin. 1989. Selective mdeium for isolation of Xanthomonas maltophilia from soil and rhizosphere environments. Appl. Environ. Microbiol. 55 : 747-750.

Kobayashi, DY, RM. Reedy, JA Bick et PV Oudemans. 2002. Characterization of a Chitinase Gene from Stenotrophomonas maltophilia Strain 34S1 and Its Involvement in Biological Control. Appl. Environ. Microbiol. 68 : 1047-1054

Kobayashi, DY., M. Guglielmoni et BB. Clarke. 1995. Isolation of the Chitinolytic Bacteria Xanthomonas maltophilia and Serratia marcescens As Biological Control Agents for Summer Patch Disease of Turfgrass. Soil Biol. Biochem. Vol.2 7. No. 1 I, Pp. 1479-1487, 1995

Klähn, S., DM. Marquardt, I. Rollwitz et M. Hagemann. 2008. Expression of the Ggpps Gene for Glucosylglycerol Biosynthesis from Azotobacter vinelandii Improves the Salt Tolerance of Arabidopsis thaliana. J. Exp. Bot. Doi: 10.1093/Jxb/Erp030, Jxb.Oxfordjournals.Org

Klähn, S, A Höhne, E Simon et M Hagemann. 2010. The Gene Ssl3076 Encodes a Protein Mediating the Salt-Induced Expression of Ggps for the Biosynthesis of the Compatible Solute Glucosylglycerol in Synechocystis Sp. Strain Pcc 6803. J. Bacteriol. 192: 4403-4412

Kobayashi, DY., RM. Reedy, JB. Bick et PV. Oudemans. 2002. Characterization of a Chitinase Gene from Stenotrophomonas maltophilia Strain 34s1 and Its Involvement in Biological Control. Appl. Environ. Microbiol., P. 1047-1054 Vol. 68, No. 3

Kurz, M. AY. Burch, B. Seip, SE. Lindow et H. Gross. 2010. Genome-Driven Investigation of Compatible Solute Biosynthesis Pathways of Pseudomonas Syringae Pv. Syringae and Their Contribution to Water Stress Tolerance. Appl. Environ. Microbiol. 76: 5452-5462

Madkour, MA., L. Tombras Smith et GM. Smith. 1990. Prefernetial osmolyte accumulation : a mechanism of osmotic stress adaptation in diazotrophic bacteria. Appl. Environ. Microbiol. 56 : 2876-2881.

Marin, K., J. Huckauf, S. Fulda et M. Hagemann. 2002. Salt-Dependent Expression of Glucosylglycerol-Phosphate Synthase, Involved in Osmolyte Synthesis in the Cyanobacterium Synechocystis Sp. Strain Pcc 6803t. J. Bacteriol. 184: 2870-2877.

Metcalfe, AC., M. Krsek, G. W. Gooday, J. I. Prosser, and E. M. H. Wellington. 2002a. Molecular Analysis of a Bacterial Chitinolytic Community in an Upland Pasture. Appl. Environ. Microbiol. 68 : 5042-5050.

Metcalfe, AC., N. Williamson, M. Krsek et EHH. Wellington. 2002b. Molecular Diversity Within Chitinolytic Actinomycetes Determined by in situ Analysis. Actinomycetol. 17:18-22

Miller, K.J. et J.M. Wood. 1996. Osmoadaptation by rhizosphere bacteria. Annu. Rev. Microbiol 50 :101-136.

Murty, MG. 1984. Phyllosphere of cotton as a habitat for diazotrophic microorganisms. Appl. Environ. Microbiol. 48: 713-718

Palleroni, N et JF Bradbury. 1993. Stenotrophomonas, a new bacteria genus for Xanthomonas maltophilia (Hugh 1980) Swings et al. 1983. Intl. J. Syst. Bacteriol. 43 : 606-609.

Pilizota T, Shaevitz JW (2012) Fast, Multiphase Volume Adaptation to Hyperosmotic Shock by Escherichia coli, PLoS ONE 7(4): e35205. doi:10.1371/journal.pone.0035205

Purvis, JE., LP. Yomano et LO Ingram. 2005. Enhanced trehalose production improves growth of Escherichia coli under osmotic stress. Appl. Environ. Microbiol. 71 : 3761-3769.

Sabaratnam, S et GA Beattie. 2003. Differences Between Pseudomonas Syringae Pv. Syringae B728a and Pantoea agglomerans Brt98 In Epiphytic and Endophytic Colonization of Leaves. Appl. Environ. Microbiol. 69 :1220-1228

Schnider-Keel, U., K. Bang Lejbolle, E. Baehler, D. Hass et C. Keel. 2001. The sigma factor AlgU (AlgT) controls exopolysaccharride production and tolerance towards osmotic stress in the biocontrol agent Pseudomonas fluorescens CHAO. Appl. Environ. Microbiol. 67 (12) : 5683-5693.

Sleator, RD et C Hill. 2001. Bacterial osmoadaptation : the role osomolytes in bacterial stress and virulence. FEMS Microbiol. Rev. 26: 49-71.

Spitzer J. 2011. From Water and Ions to Crowded Biomacromolecules: In Vivo Structuring of a Prokaryotic Cell. Microbiol Molec. Biol. Rev. 75(3): 491-506

Wolf, A., A. Fritze, M. Hagemann et G. Berg. 2002. Stenotrophomonas rhizophila Sp. Nov., a Novel Plant-Associated Bacterium with Antifungal Properties. Intl. J. Syst. Evol. Microbiol. 52 : 1937-1944

Wood, JM. 1999. Osmosensing by bacteria : signals and membrance - based sensors. MMBR 63 : 230.

Yoon, J-H, S-J Kang, HW Oh et T-K Oh. 2008. Stenotrophomonas dokdonensis Sp. Nov., Isolated from Soil. Intl. J. Syst. Evol. Microbiol. 56, 1363-1367

Zimmerman, SB. et SO Trach. 1991. Estimation of macromolecule concentration and excluded volunme effects for the cytoplasm of Escherichia coli. J. Mol. Biol. 222 : 599-620.

## Revendications

1. Procédé de préparation de bouillies inoculantes pulvérisables comprenant des populations bactériennes osmotolérantes **caractérisé en ce que** les bouillies sont réalisées en deux temps ;

   (i) création dans un premier temps de pré - bouillies plus concentrées en sels fertilisants que les susdites bouillies par un facteur de concentration permettant d'atteindre une milliosmolarité (mOSM) critique responsable de l'induction statique de l'osmotolérance des susdites populations bactériennes a *priori* osmotolérantes, et
   (ii) abolition dans une deuxième temps et après une certaine période d'induction statique de ce facteur de concentration par dilution desdites pré - bouillies jusqu'à un volume correspondant au volume préconisé pour de telles bouillies pulvérisables contenant des populations osmotolérantes.

2. Procédé de préparation de bouillies inoculantes pulvérisables comprenant des populations bactériennes osmotolérantes selon la première revendication **caractérisé en ce que** les populations bactériennes sont constituées d'espèces bactériennes Gram négatives (Gram⁻).

3. Procédé de préparation de bouillies inoculantes pulvérisables comprenant des populations bactériennes osmotolérantes selon la première revendication **caractérisé en ce que** les populations bactériennes sont constituées d'espèces bactériennes Gram positives (Gram⁺).

4. Procédé de préparation de bouillies inoculantes pulvérisables comprenant des populations bactériennes osmotolérantes selon une quelconque des revendications précédentes **caractérisé en ce que** la milliosmolarité (mOSM) critique de la pré - bouillie concentrée est comprise entre 330 et 1 250, et avantageusement aux alentours de 500.

5. Procédé de préparation de bouillies inoculantes pulvérisables comprenant des populations bactériennes osmotolérantes selon une quelconque des revendications précédentes **caractérisé en ce que** la période d'*induction statique* est comprise entre 6 et 24 heures, plus particulièrement entre 8 et 18 heures, et plus usuellement d'environs 12 heures.

6. Procédé de préparation de bouillies inoculantes pulvérisables comprenant des populations bactériennes osmotolérantes selon une quelconque des revendications précédentes **caractérisé en ce que** la dilution des pré - bouillies est effectuée par l'apport d'un volume aqueux fonction de la dose hectare (L/ha) du produit, du poids moléculaire des sels fertilisants le constituant (g/mole), et leurs concentrations dans le produit (g-sel/L) respectant une mOSM minimale de la bouillie fonction de l'osmolarité de croissance (OSMc) appliquée lors production des populations bactériennes en milieux liquides et/ou solide.

7. Procédé de préparation de bouillies inoculantes pulvérisables comprenant des populations bactériennes osmotolérantes selon la revendication précédente **caractérisé en ce que** la mOSM minimale fonction de l'osmolarité de croissance (OSMc) appliquée lors production des populations bactériennes en milieux liquides et/ou solide est elle fonction de l'osmolarité de plasmolyse (OSMp) de ces populations bactériennes.

8.  Procédé de préparation de bouillies inoculantes pulvérisables comprenant des populations bactériennes osmotolérantes selon la revendication précédente **caractérisé en ce que** l'osmolarité de plasmolyse (OSMp) de ces populations bactériennes est elle donnée par la le paramètre $\alpha_2$ (mOSM) de la relation décrivant le volume cellulaire, $V_c$ (uL/mg de protéine bactérienne), desdites bactéries en fonction d'OSMPp, à savoir **[$V_c = \alpha_1 / (OSMp + \alpha_2) + V_{c,min}$,] où $V_{c,min}$** et le volume cellulaire minimal atteint en fonction d'OSMp et $\alpha_1$ est un autre paramètre empirique (uL/mg de protéine bactérienne).

9.  Procédé de préparation de bouillies inoculantes pulvérisables comprenant des populations bactériennes osmotolérantes selon une quelconque des revendications précédentes **caractérisé en ce que** les populations bactériennes *a priori* osmotolérantes sont produites par fermentation liquide et/ou solide en présence de milli - osmolarités de croissance (OSMc) comprises entre 50 et 1 000, plus particulièrement entre 200 et 800, et avantageusement entre 300 et 600.

10. Procédé de préparation de bouillies inoculantes pulvérisables comprenant des populations bactériennes osmotolérantes selon une quelconque des revendications précédentes **caractérisé en ce que** le facteur de concentration à abolir est compris entre 3,8 et 113 et cela selon la concentration (g-sel/L) des sels fertilisant, le poids moléculaire (PM) de ceux-ci et la quantité de produit préconisé par hectare (i.e. dose-hectare en g, Kg ou L par hectare), voire - le cas échéant, l'osmolarité de la fraction soluble de la formulation solide et/ou liquide, de ladite préparation bactérienne.

11. Procédé de préparation de bouillies inoculantes pulvérisables comprenant des populations bactériennes osmotolérantes selon la revendication précédente **caractérisé en ce que** la milliosmolarité (mOSM) de la bouillie une fois ledit facteur de concentration aboli par dilution aqueuse ne doit pas elle être inférieure à des valeurs comprises entre 175 et 360, plus avantageusement entre 190 et 330, notamment dans le cas de bactéries produites en présence d'OSMc (omolarités de croissance) les plus élevées, soit ici de l'ordre de 1,00.

12. Procédé de préparation de bouillies inoculantes pulvérisables comprenant des populations bactériennes osmotolérantes selon une quelconque des revendications précédentes **caractérisé en ce que** les populations bactériennes sont chitinolytiques.

13. Procédé de préparation de bouillies inoculantes pulvérisables comprenant des populations bactériennes osmotolérantes selon une quelconque des revendications précédentes **caractérisé en ce que** les populations bactériennes *a priori* osmotolérantes proviennent de familles taxonomiques choisies parmi un groupe comprenant les *Xanthomonadaceae* et les *Pseudomonadaceae.*

14. Procédé de préparation de bouillies inoculantes pulvérisables comprenant des populations bactériennes osmotolérantes selon la revendication précédente **caractérisé en ce que** les populations bactériennes *a priori* osmotolérantes proviennent du genre *Stenotrophomonas.*

15. Procédé de préparation de bouillies inoculantes pulvérisables comprenant des populations bactériennes osmotolérantes selon une quelconque des deux revendications précédentes **caractérisé en ce que** les populations bactériennes a *priori* osmotolérantes produisent comme osmolyte compatible du glucosyl-glycérol (GG) et/ou du glucosyl-glycerate (GGate).

Figure 1

**Figure 2**

PM : 113

PM : 90

PM : 80

Figure 3

Figure 4 :

Figure 5

Figure 6

Figure 7

Europäisches
Patentamt

European
Patent Office

Office européen
des brevets

**RAPPORT DE RECHERCHE EUROPEENNE**

Numéro de la demande

EP 13 36 6002

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| A,D | BONATERRA ANNA ET AL: "Increasing survival and efficacy of a bacterial biocontrol agent of fire blight of rosaceous plants by means of osmoadaptation", FEMS MICROBIOLOGY ECOLOGY, vol. 61, no. 1, juillet 2007 (2007-07), pages 185-195, XP002667638, ISSN: 0168-6496 * le document en entier * | 1-15 | INV. C12N1/20 C05F11/08 A01N63/02 C05G3/00 |
| A,D | BONATERRA A ET AL: "Osmotically induced trehalose and glycine betaine accumulation improves tolerance to desiccation, survival and efficacy of the postharvest biocontrol agent Pantoea agglomerans EPS125", FEMS MICROBIOLOGY LETTERS, WILEY-BLACKWELL PUBLISHING LTD, GB, vol. 250, no. 1, 1 septembre 2005 (2005-09-01), pages 1-8, XP027871909, ISSN: 0378-1097 [extrait le 2005-09-01] * le document en entier * | 1-15 | |
| A | FR 2 880 344 A1 (CLAUDE PIERRE PHILIPPE [FR]) 7 juillet 2006 (2006-07-07) * page 9 - page 10 * | 1-15 | **DOMAINES TECHNIQUES RECHERCHES (IPC)** C12N C05F A01N C05G |

-/--

Le présent rapport a été établi pour toutes les revendications

| Lieu de la recherche | Date d'achèvement de la recherche | Examinateur |
|---|---|---|
| La Haye | 12 juillet 2013 | Lejeune, Robert |

CATEGORIE DES DOCUMENTS CITES

X : particulièrement pertinent à lui seul
Y : particulièrement pertinent en combinaison avec un
   autre document de la même catégorie
A : arrière-plan technologique
O : divulgation non-écrite
P : document intercalaire

T : théorie ou principe à la base de l'invention
E : document de brevet antérieur, mais publié à la
   date de dépôt ou après cette date
D : cité dans la demande
L : cité pour d'autres raisons

& : membre de la même famille, document correspondant

EPO FORM 1503 03.82 (P04C02)

Europäisches Patentamt
European Patent Office
Office européen des brevets

## RAPPORT DE RECHERCHE EUROPEENNE

Numéro de la demande
EP 13 36 6002

### DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| A | Dr. Krishan Chandra et al.: "Liquid biofertilizers", Liquid Biofertilizers and Biocontrol Agents , mars 2005 (2005-03), pages FRONTPG.-3,16-65, XP002667639, Extrait de l'Internet: URL:http://www.liquidbiofertilizers.com/liquid.pdf [extrait le 2012-01-19] * page 56 * ----- | 1-15 | |
| A | FNCA Biofertilizer Project Group: "Section 2 of Chapter IV: Non-symbiotic nitrogen fixers", Forum for Nuclear Cooperation in Asia (FNCA) Biofertilizer Manual (http://www.fnca.mext.go.jp/bf/bfm/pdf/Biofertilizer_Manual.pdf), 2006, pages 69-89, XP002667640, Extrait de l'Internet: URL:http://www.fnca.mext.go.jp/bf/bfm/pdf/4_2_Non-Symbiotic_Nitrogen_Fixers0404.pdf [extrait le 2012-01-19] * page 89 * ----- | 1-15 | DOMAINES TECHNIQUES RECHERCHES (IPC) |
| A | EP 1 402 779 A1 (UNI DE GIRONA [ES]) 31 mars 2004 (2004-03-31) * alinéa [0040] * ----- -/-- | 1-15 | |

Le présent rapport a été établi pour toutes les revendications

1

| Lieu de la recherche | Date d'achèvement de la recherche | Examinateur |
|---|---|---|
| La Haye | 12 juillet 2013 | Lejeune, Robert |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

Europäisches
Patentamt

European
Patent Office

Office européen
des brevets

**RAPPORT DE RECHERCHE EUROPEENNE**

Numéro de la demande

EP 13 36 6002

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| A | RODER A ET AL: "Synthesis of the compatible solutes glucosylglycerol and trehalose by salt-stressed cells of Stenotrophomonas strains", FEMS MICROBIOLOGY LETTERS, vol. 243, no. 1, 1 février 2005 (2005-02-01), pages 219-226, XP004724621, BLACKWELL PUBLISHING, AMSTERDAM, NL ISSN: 0378-1097, DOI: 10.1016/J.FEMSLE.2004.12.005 * le document en entier * ----- | 1-15 | |

DOMAINES TECHNIQUES RECHERCHES (IPC)

Le présent rapport a été établi pour toutes les revendications

| Lieu de la recherche | Date d'achèvement de la recherche | Examinateur |
|---|---|---|
| La Haye | 12 juillet 2013 | Lejeune, Robert |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

**ANNEXE AU RAPPORT DE RECHERCHE EUROPEENNE
RELATIF A LA DEMANDE DE BREVET EUROPEEN NO.**

EP 13 36 6002

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

12-07-2013

| Document brevet cité au rapport de recherche | | Date de publication | Membre(s) de la famille de brevet(s) | | Date de publication |
|---|---|---|---|---|---|
| FR 2880344 | A1 | 07-07-2006 | CA | 2491845 A1 | 04-07-2006 |
| | | | FR | 2880344 A1 | 07-07-2006 |
| | | | FR | 2886944 A1 | 15-12-2006 |
| EP 1402779 | A1 | 31-03-2004 | AT | 382263 T | 15-01-2008 |
| | | | EP | 1402779 A1 | 31-03-2004 |
| | | | ES | 2188375 A1 | 16-06-2003 |
| | | | WO | 02098233 A1 | 12-12-2002 |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 10366001 A

- FR 0900867

**Littérature non-brevet citée dans la description**

- **BOLLET, C. ; A. DAVIN-REGLI ; P. DE MICCO.** A Simple Method for Selective Isolation of Stenotrophomonas maltophilia from Environmental Samples. *Appl. Environ. Microbiol.,* 1995, vol. 61, 1653-54
- **BONATERRA, A ; J CAMPS ; E MONTESINOS.** Osmotically induced trehalose and glycine betaine accumulation improves tolerance to desiccation, survival and e☐cacy of the postharvest biocontrol agent Pantoea agglomerans EPS125. *FEMS Microbiol. Lett.,* 2005, vol. 250, 1-8
- **BONATERRA, A. ; J. CABREFIGA ; J. CAMPS ; E. MONTESINOS.** Increasing survfival and efficacy of bacterial biocontrol agent of fire bligth of rosaceaous plants by meands of osmoadaptation. *FEMS Microbiol. Ecol.,* 2007, vol. 61, 185-195
- **CAYLEY, DS. ; HJ. GUTTMAN ; MT. RECORD, JR.** Biophysical characterization of changes in amounts and activity of E. coli cell and compartment water and turgor pressure in response to osmotic stress. *Biophycisal. J.,* 2000, vol. 78, 1748-1764
- **CAYLEY, DS ; BA. LEWIS ; HJ. GUTTMAN ; MT. RECORD, JR.** Characterization of the cytoplasm of Escherichia coli K-12 as a function of external osmolarity : implications for protein - DNA interactions in vivo. *J. Mol. Biol.,* 1991, vol. 222, 281-300
- **CROSSMAN, LC. ; VC GOULD ; JM DOW ; GS VERNIKOS ; A OKAZAKI ; M SEBAIHIA ; D SAUNDERS ; C ARROWSMITH ; T CARVER ; NP, E ADLEM.** The Complete Genome, Comparative and Functional Analysis of Stenotrophomonas maltophilia Reveals an Organism Heavily Shielded by Drug Resistance Determinants. *Genome Biology,* 2008, vol. 9, R74
- **CSONKA, L.N.** Physiological and genetic responses of bacteria to osmotic stress. *Microbiol. Rev.,* 1989, vol. 53, 121-147
- **DÔTSCH, A. ; J. SEVERIN ; W. ALT ; EA. GALINSKI ; J-U. KREFT.** A mathematical model for growth and osmoregulation in halophilic bacteria. *Microbiology,* 2008, vol. 154, 2956-2969
- **ELLIS, RJ.** Macromolecular crowding : an important but neglected aspect of the intracellular environment. *Curr. Op. Struct. Biol.,* 2001, vol. 11, 114-119

- **FERJANI, A. ; L. MUSTARDY ; R. SULPICE ; K. MARIN ; I. SUZUKI ; M. HAGEMANN ; N. MURATA.** Glucosylglycerol, a Compatible Solute, Sustains Cell Division Under Salt Stress. *Plant Physiol.,* 2003, vol. 131, 1628-1637
- **GIESLER, LJ. ; GY. YUEN.** Evaluation of Stenotrophomonas maltophilia Strain C3 for Biocontrol of Brown Patch Disease. *Crop Protect.,* 1998, vol. 17, 509-513
- **GOLDMAN. S. ; K. HECHT ; H. EISENBERG ; M. MEVARECH.** Extracellular Ca2+-Dependent Inducible Alkaline Phosphatase from the Extremely Halophilic Archaebacterium Haloarcula marismortui. *J. Bacteriol.,* 1990, vol. 172, 7065-70
- **GOUDE, R. ; S. RENAUD ; S. BONNASSIE ; T. BERNARD ; C. BLANCO.** Glutamine, Glutamate, and☐-Glucosylglycerate are the Major Osmotic Solutes Accumulated by Erwinia chrysanthemi Strain 3937. *Appl. Environ. Microbiol.,* 2004, vol. 70, 6535-6541
- **GOUESBET, G ; A TRAUTWETTER ; S BONNASSIE ; LF WU ; C BLANCO.** Characterization of the Erwinia chrysanthemi osmoprotectant transporter gene ousA. *J. Bacteriol.,* 1996, vol. 78, 447-455
- **HAGEMANN, M. ; K. RIBBECK-BUSCH ; S. KLÄHN ; D. HASSE ; R. STEINBRUCH ; G. BERG.** The Plant-Associated Bacterium Stenotrophomonas Rhizophila Expresses a New Enzyme for the Synthesis of the Compatible Solute Glucosylglycerol. *J. Bacteriol.,* 2008, vol. 190, 5898-5906
- **HAGEMANN, M. ; N. ERDMANN.** Activation and Pathway of glucosyl glycerol Synthesis in the Cyanobacterium Synechocystis Sp. Pcc 6803. *Microbiology,* 1994, vol. 140, 1427-1431
- **HEYLEN, K. ; B. VANPARYS ; F. PEIRSEGAELE ; L. LEBBE ; P. DE VOS.** Stenotrophomonas Terrae Sp. Nov. and Stenotrophomonas Humi Sp. Nov., Two Nitrate-Reducing Bacteria Isolated from Soil. *Intl. J. Syst. Evol. Microbiol.,* 2007, vol. 57, 2056-2061

- **JOHAN H. ; J. LEVEAU ; STEVEN E. LINDOW.** Appetite of An Epiphyte: Quantitative Monitoring of Bacterial Sugar Consumption In The Phyllosphere. *Pnas,* 2001, vol. 98 (6), 3446-3453
- **JUHNKE, ME ; E DES JARDIN.** Selective mdeium for isolation of Xanthomonas maltophilia from soil and rhizosphere environments. *Appl. Environ. Microbiol.,* 1989, vol. 55, 747-750
- **KOBAYASHI, DY ; RM. REEDY ; JA BICK ; PV OUDEMANS.** Characterization of a Chitinase Gene from Stenotrophomonas maltophilia Strain 34S1 and Its Involvement in Biological Control. *Appl. Environ. Microbiol.,* 2002, vol. 68, 1047-1054
- **KOBAYASHI, DY. ; M. GUGLIELMONI ; BB. CLARKE.** Isolation of the Chitinolytic Bacteria Xanthomonas maltophilia and Serratia marcescens As Biological Control Agents for Summer Patch Disease of Turfgrass. *Soil Biol. Biochem.,* 1995, vol. 2 7 (1 I), 1479-1487
- **KLÄHN, S. ; DM. MARQUARDT ; I. ROLLWITZ ; M. HAGEMANN.** Expression of the Ggpps Gene for Glucosylglycerol Biosynthesis from Azotobacter vinelandii Improves the Salt Tolerance of Arabidopsis thaliana. *J. Exp. Bot.,* 2008
- **KLÄHN, S ; A HÖHNE ; E SIMON ; M HAGEMANN.** The Gene Ssl3076 Encodes a Protein Mediating the Salt-Induced Expression of Ggps for the Biosynthesis of the Compatible Solute Glucosylglycerol in Synechocystis Sp. Strain Pcc 6803. *J. Bacteriol.,* 2010, vol. 192, 4403-4412
- **KOBAYASHI, DY. ; RM. REEDY ; JB. BICK ; PV. OUDEMANS.** Characterization of a Chitinase Gene from Stenotrophomonas maltophilia Strain 34s1 and Its Involvement in Biological Control. *Appl. Environ. Microbiol.,* 2002, vol. 68 (3), 1047-1054
- **KURZ, M ; AY. BURCH ; B. SEIP ; SE. LINDOW ; H. GROSS.** Genome-Driven Investigation of Compatible Solute Biosynthesis Pathways of Pseudomonas Syringae Pv. Syringae and Their Contribution to Water Stress Tolerance. *Appl. Environ. Microbiol.,* 2010, vol. 76, 5452-5462
- **MADKOUR, MA. ; L. TOMBRAS SMITH ; GM. SMITH.** Prefernetial osmolyte accumulation : a mechanism of osmotic stress adaptation in diazotrophic bacteria. *Appl. Environ. Microbiol.,* 1990, vol. 56, 2876-2881
- **MARIN, K. ; J. HUCKAUF ; S. FULDA ; M. HAGEMANN.** Salt-Dependent Expression of Glucosylglycerol-Phosphate Synthase, Involved in Osmolyte Synthesis in the Cyanobacterium Synechocystis Sp. Strain Pcc 6803t. *J. Bacteriol.,* 2002, vol. 184, 2870-2877
- **METCALFE, AC. ; M. KRSEK ; G. W. GOODAY ; J. I. PROSSER ; E. M. H. WELLINGTON.** Molecular Analysis of a Bacterial Chitinolytic Community in an Upland Pasture. *Appl. Environ. Microbiol.,* 2002, vol. 68, 5042-5050
- **METCALFE, AC. ; N. WILLIAMSON ; M. KRSEK ; EHH. WELLINGTON.** Molecular Diversity Within Chitinolytic Actinomycetes Determined by in situ Analysis. *Actinomycetol,* 2002, vol. 17, 18-22
- **MILLER, K.J. ; J.M. WOOD.** Osmoadaptation by rhizosphere bacteria. *Annu. Rev. Microbiol,* 1996, vol. 50, 101-136
- **MURTY, MG.** Phyllosphere of cotton as a habitat for diazotrophic microorganisms. *Appl. Environ. Microbiol.,* 1984, vol. 48, 713-718
- **PALLERONI, N ; JF BRADBURY.** *Stenotrophomonas, a new bacteria genus for Xanthomonas maltophilia,* 1993
- **SWINGS et al.** *Intl. J. Syst. Bacteriol.,* 1983, vol. 43, 606-609
- **PILIZOTA T ; SHAEVITZ JW.** Fast, Multiphase Volume Adaptation to Hyperosmotic Shock by Escherichia coli. *PLoS ONE,* 2012, vol. 7 (4), e35205
- **PURVIS, JE. ; LP. YOMANO ; LO INGRAM.** Enhanced trehalose production improves growth of Escherichia coli under osmotic stress. *Appl. Environ. Microbiol.,* 2005, vol. 71, 3761-3769
- **SABARATNAM, S ; GA BEATTIE.** Differences Between Pseudomonas Syringae Pv. Syringae B728a and Pantoea agglomerans Brt98 In Epiphytic and Endophytic Colonization of Leaves. *Appl. Environ. Microbiol.,* 2003, vol. 69, 1220-1228
- **SCHNIDER-KEEL, U. ; K. BANG LEJBOLLE ; E. BAEHLER ; D. HASS ; C. KEEL.** The sigma factor AlgU (AlgT) controls exopolysaccharide production and tolerance towards osmotic stress in the biocontrol agent Pseudomonas fluorescens CHAO. *Appl. Environ. Microbiol.,* 2001, vol. 67 (12), 5683-5693
- **SLEATOR, RD ; C HILL.** Bacterial osmoadaptation : the role osomolytes in bacterial stress and virulence. *FEMS Microbiol. Rev.,* 2001, vol. 26, 49-71
- **SPITZER J.** From Water and Ions to Crowded Biomacromolecules: In Vivo Structuring of a Prokaryotic Cell. *Microbiol Molec. Biol. Rev.,* 2011, vol. 75 (3), 491-506
- **WOLF, A. ; A. FRITZE ; M. HAGEMANN ; G. BERG.** Stenotrophomonas rhizophila Sp. Nov., a Novel Plant-Associated Bacterium with Antifungal Properties. *Intl. J. Syst. Evol. Microbiol.,* 2002, vol. 52, 1937-1944
- **WOOD, JM.** Osmosensing by bacteria : signals and membrane - based sensors. *MMBR,* 1999, vol. 63, 230
- **YOON, J-H ; S-J KANG ; HW OH ; T-K OH.** Stenotrophomonas dokdonensis Sp. *Soil. Intl. J. Syst. Evol. Microbiol.,* Novembre 2008, vol. 56, 1363-1367
- **ZIMMERMAN, SB. ; SO TRACH.** Estimation of macromolecule concentration and excluded volunme effects fo r the cytoplasm of Escherichia coli. *J. Mol. Biol.,* 1991, vol. 222, 599-620